# EUROPÄISCHE PATENTANMELDUNG

(11) **EP 3 178 463 A1**
(43) Veröffentlichungstag der Anmeldung: **14.06.2017**
(21) Anmeldenummer: 16169371.8
(22) Anmeldetag: 12.05.2016
(51) Int. Cl.: A61K 6/02, C04B 41/00, C04B 41/45, C04B 41/89, C04B 41/52, A61C 13/083, B05C 1/00, A61C 13/00, A61K 6/00, C04B 111/00, C04B 111/82

(54) **VERFAHREN ZUR HERSTELLUNG EINES KERAMISCHEN KÖRPERS, INSBESONDERE EINES DENTALKERAMIKROHLINGS, MIT RÄUMLICH GEZIELT EINSTELLBAREN AUSPRÄGUNGSGRADEN PHYSIKALISCHER EIGENSCHAFTEN**

(30) Priorität: 07.12.2015 DE 102015121246
(71) Anmelder: WDT-Wolz-Dental-Technik GmbH, 55566 Bad Sobernheim (DE)
(72) Erfinder: Wolz, Stefan, 55566 Bad Sobernheim (DE)
(74) Vertreter: Götz, Georg Alois

(57) **Zusammenfassung**

Verfahren zur Herstellung eines keramischen Körpers (100), insbesondere eines Dentalkeramikrohlings, mit gezielt einstellbaren Ausprägungsgraden einer oder unterschiedlicher physikalischer Eigenschaften, wobei der keramische Körper (100) zur Steuerung einer gezielten Verteilung eines oder verschiedener chemischer Stoffe (101, 102), die zur Beeinflussung der physikalischen Eigenschaften des keramischen Körpers (100) geeignet sind, eine Porosität aufweist, und in einem ersten Schritt, der ein Beladungsschritt ist, mit einer oder mit mehreren Lösungen (104) des einen oder der verschiedenen chemischen Stoffe (101, 102) beladen wird. In einem zweiten Schritt, der ein Verteilungssteuerungsschritt ist, wird die Verteilung des einen oder der verschiedenen chemischen Stoffe (101, 102) innerhalb des porösen, keramischen Körpers (100) gesteuert, wobei ein Verlauf und/oder ein räumlicher Verlauf des Ausprägungsgrads der einen oder der unterschiedlichen physikalischen Eigenschaften einstellbar ist. Die Steuerung erfolgt durch Regelung eines oder mehrerer Umgebungsparameter (106) in einer Umgebung (108), insbesondere durch Regelung der Luftfeuchtigkeit und/oder des Drucks und/oder der Temperatur.

## Beschreibung

Die Erfindung betrifft ein Verfahren zur Herstellung eines keramischen Körpers, insbesondere eines polychrom, monochrom oder räumlich gefärbten Dentalkeramikrohlings, mit gezielt einstellbaren Ausprägungsgraden einer oder unterschiedlicher physikalischer Eigenschaften. Vorzugsweise zeigen die eine oder die unterschiedlichen physikalischen Eigenschaften unterschiedliche Ausprägungsgrade bezüglich räumlich verschiedener Bereiche des keramischen Körpers bzw. weist der keramische Körper einen flächigen, d. h. zweidimensionalen und/oder räumlichen, d.h. dreidimensionalen Verlauf der Ausprägungsgrade der einen oder der unterschiedlichen physikalischen Eigenschaften auf. Unter den physikalischen Eigenschaften sind beispielsweise optische Eigenschaften, wie Opazität und Transluzenz, mechanische Eigenschaften, wie Härte, (Biege-)Festigkeit, und (Bruch-)Zähigkeit, aber auch strukturelle Eigenschaften, wie Kristallsystemkonfigurationen, Dichte und/oder Porosität usw. zu verstehen.

In den letzten Jahren hat sich in der Dentaltechnik im Bereich der Vollkeramik sogenanntes Yttrium stabilisiertes Zirkonoxid, insbesondere teilstabilisiertes Zirkonoxid mit etwa 3%-Molanteil an Yttriumoxid, (3Y-TZP = Yttria-Tetragonal Zirconia Polycristals) durchgesetzt. Die ausschlaggebenden Gründe dafür sind zum einen die hohe mechanische Stabilität und thermische Beständigkeit, aber auch die exzellente Biokompatibilität dieser Hochleistungskeramikgerüste. Üblicherweise erfolgt eine abschließende Farbgebung bei vollkeramischem Zahnersatz durch eine Verblendung, d. h. das Aufbringen einer zusätzlichen Keramikschicht auf den bereits in Form gefrästen Zahnersatz, bspw. Kronen oder Brücken. Die zusätzliche Keramikschicht, die hervorragende Farb- und Formgebungsmöglichkeiten besitzt, wird heute noch aufwendig und individuell für jeden einzelnen Zahnersatz von Hand aufgebracht. Jedoch wurde anhand von Studien nachgewiesen, dass diese ästhetische Verblendkeramik eine fünfmal höhere Abplatzungsrate der Verblendung, als die bewährte Verblendmetallkeramik (VMK) aufweist. Auf Grund von Reklamationen und der hohen Herstellungskosten soll auf die ästhetische Verblendkeramik, verzichtet werden. Ohne keramische Verblendung weist die Yttrium stabilisierte Zirkonoxid-Vollkeramik dann jedoch eine doppelt so hohe Härte wie die einer natürlichen Zahnkrone auf, was zum Verschleiß gegenüberliegender und benachbarter Zähne führt und so den Rest des Zahnbestandes schädigen kann.

Ein zusätzliches Problem, das sich aus dem Verzicht auf eine keramische Verblendung ergibt, ist die Optik bzw. die Ästhetik des Zahnersatzes. Hier sei anzumerken, dass es keinen einfarbigen Zahn im Mund eines Patienten gibt. Bei einem natürlichen Zahn wird der dunklere Dentinkern von immer dicker werdenden Schichten an Schneidezahnmasse überzogen. Dies führt zu unterschiedlichen Farbgebungen und Lichttransmissionseigenschaften des natürlichen Zahns, bis hin zu nahezu transparenten Schneidkanten. Ein herkömmlicher, nicht verblendeter keramischer Zahnersatz, insbesondere aus reinem 3Y-TZP weist eine räumlich einheitliche Ausprägung farblicher und physikalischer Eigenschaften, insbesondere der Härte, aber auch der Lichttransmissionseigenschaften wie Transluzenz bzw. Opazität auf.

Damit eine Krone bzw. der Zahnersatz ästhetisch wirkt, sollten die im nachfolgenden aufgeführten Merkmale berücksichtigt und umgesetzt werden. Zum einen muss hierfür die Dentinschicht die Grundzahnfarbe des Patienten aufweisen. Die Dentinfarbe und die Schneideschichtstärke bilden die eigentliche Zahnfarbe des Patienten. Die dreidimensionale Farbkombination bildet der natürliche Zahn im Wachstum oder durch spätere Abnutzung. Zum anderen gibt es auch einen immer heller werdenden Farbverlauf vom Dentinkern bis hin zur Schneide. Der Zahnschmelz kann außerdem teilweise helle und/oder transparente Stellen aufweisen. Des Weiteren sind bei älteren Patienten die dunklen dentinfarbenen Kronenränder gut sichtbar, so dass der neue Zahnersatz dementsprechend angepasst werden muss.

Gemäß WO 2008 098 157, WO 2013 055 432, WO 0046 168, WO 2004 110 959,DE 199 04 522 B4, DE 10 2008 026 980 A1, WO 00/46168 A1, WO 2011/156602 A2 oder DE 20 2011 109 956 U1, WO 11 15 66 02, EP 2013 06 31 20, US 2011 039 805 sind verschiedene Verfahren zum Einfärben von Zahnersatz mit Metallionen und/oder Metallkomplexlösungen und -gelen bekannt. Durch das Eintauchen oder Besprühen eines Zahnersatzes kann jedoch maximal eine einfache und einfarbige Färbung erreicht werden. Es wird zwar versucht mittels eines Pinsels oder Tropfenapplikationen Zahnfärbungsstrukturen oder ähnliche Einfärbungen auf dem Zahnersatz zu erzielen, jedoch lässt sich mit den bekannten Flüssigkeiten bzw. Lösungen oder Gelen kein naturidentisches Ergebnis erzielen, sodass sich zwischen natürlichen Zähnen und Zahnersatz merkliche, farbliche Unterschiede ergeben.

Um den Zahnersatz nicht manuell individualisiert, sondern automatisiert herzustellen, müsste z.B. eine Vielzahl von ganz unterschiedlichen Keramikrohlingen mit räumlichen Farbverläufen aber auch räumlichen Verläufen der physikalischen Eigenschaften hergestellt werden, um kostengünstige und ästhetische Ergebnisse zu erreichen.

Die EP 202 4300, WO 2014 062 375, WO 02 09 6 12, US 92 12 065 B2, DE 2020 090 187 24, EP 235 97 71 und EP 185 97 57 lehren das Einfärben des Ausgangsmaterials, insbesondere von Pulvern oder Pasten. Die Pulver oder Pasten werden dann schichtweise geschüttet bzw. aufgetragen, wobei jede Schicht eine konkrete Farbe aufweist. Somit sind 7-10 Schichten erforderlich, um eine zweidimensionale Farbgebung bzw. einen zweidimensionalen oder flächigen Farbverlauf zu erreichen.

Aus der US 2011 269 618 ist ein Verfahren zur Herstellung eines keramischen Zahnersatzes mit erhöhter optischer Transluzenz bekannt. Das Ausgangsmaterial, tetragonal polykristallines Zirkoniumpulver wird hierzu mit einer besonders geringen Partikelgröße ausgebildet. Aufgrund der geringeren Partikelgröße weist der daraus gepresste Zahnersatz einen niedrigeren Brechungsindex und folglich eine erhöhte Transluzenz auf. Gemäß der US 2013 022 15 54 A1 lässt sich zumindest ein grob gestufter Verlauf der optisch physikalischen Eigenschaften wie Opazität und/oder Transluzenz, ähnlich wie bei der Farbgebung eines Dentalkeramikrohlings, durch das schichtweise Schütten verschiedener Dentalkeramikpulver mit unterschiedlichen Anteilen an Yttrium erzielen. Der gepresste Dentalkeramikrohling weist dann einen grob gestuften, zweidimensionalen Opazitätsverlauf auf, der der Anzahl und Dicke der Schichten entspricht.

Nachteilig an den zuvor beschriebenen Verfahren ist einerseits, dass sich auf die genannte Weise lediglich ein flächiger, d.h. zweidimensionaler Farbverlauf und/oder Verlauf des Ausprägungsgrads der Transluzenz erzielen lässt. Andererseits erfordert die Ausbildung jeder weiteren Abstufung des Farb- und/oder Ausprägungsverlaufs jeweils einen zusätzlichen Verfahrensschritt. Die Erzeugung eines dreidimensionalen bzw. räumlichen sowie fein gestuften Verlaufs würde das Schütten einer Vielzahl von Pulverschichten nicht nur übereinander sondern auch nebeneinander erfordern, was den Rahmen des beschriebenen Verfahrens sprengen und zu unverhältnismäßigen Mehrkosten führen würde. Unter anderem müssten Hunderte verschiedener Pulver mit unterschiedlichen physikalischen Eigenschaften und Farbgebungen gelagert, geprüft und kontrolliert werden.

Ein aufwendiges Sol-Gel-Verfahren zur Herstellung eines fräsbaren Dentalkeramikrohlings mit geometrisch definierten Bereichen unterschiedlich ausgeprägter Transluzenz ist aus der US 2015 028 2905 A1 bekannt. Hierin wird ein erstes Zirkonium-Sol in eine Form gegossen und zu einem ersten Zirkonium-Gel gehärtet. Das erste Zirkonium Gel wird in einer zweiten, größeren Form platziert und mit einem zweiten Zirkonium-Sol übergossen. Der Vorgang wird wiederholt, bis die gewünschte Anzahl an Zirkonium-Gelschichten erreicht ist. Der geschichtete Zirkonium Gelkörper wird anschließend angesintert, um einen fräsbaren Dentalkeramikrohling zu erhalten. Jede Zirkonium-Schicht kann hierbei eine andere Transluzenz aufweisen. Zwar können mit dem beschriebenen Verfahren auch räumlich unterschiedliche Ausprägungsgrade der Transluzenz innerhalb des Dentalkeramikrohling erzielt werden, jedoch erfordert jeder zusätzliche Bereich einen zusätzlichen Verfahrensschritt, der das Gießen des Zirkonium-Sols sowie das anschließende Aushärten zum Zirkonium-Gel beinhaltet, wodurch das Verfahren äußerst zeitaufwendig und folglich unrentabel wäre.

Es ist daher Aufgabe der Erfindung, ein verbessertes, automatisierbares und daher kostengünstigeres Verfahren zur Herstellung eines keramischen Körpers, insbesondere eines Dentalkeramikrohlings, mit einem Verlauf und/oder räumlichen Verlauf der Ausprägungsgrade einer oder verschiedener physikalischer Eigenschaften anzugeben. Insbesondere ist es Aufgabe der Erfindung, ein solches Verfahren gleichzeitig zur Einstellung eines monochromen, polychromen und oder räumlich polychromen Farbverlaufs zu verwenden.

### [I|GG1]

Die Aufgabe wird durch ein Verfahren gemäß Anspruch 1 sowie durch einen Keramikrohling, der insbesondere nach einem erfindungsgemäßen Verfahren hergestellt wurde gelöst.

Vorteilhafte, optionale Ausbildungen und/oder Weiterbildungen ergeben sich ganz oder teilweise aus den abhängigen Ansprüchen.

Ein erfindungsgemäßes Verfahren der eingangs beschriebenen Art kennzeichnet sich dadurch, dass der keramische Körper zur Steuerung einer gezielten Verteilung eines oder verschiedener chemischer Stoffe, die zur Beeinflussung der physikalischen Eigenschaften des keramischen Körpers geeignet sind, eine Porosität aufweist. Unter einer porösen Keramik, insbesondere in der Dentaltechnik, versteht man im Allgemeinen einen porösen, keramischen Körper der zum Beispiel durch Pressen aus Keramikpulver, durch Schlickergießen aus keramischem Schlicker, mittels eines Keramik-3D-Druckers oder durch ähnliche, geeignete Verfahren geformt wurde. Insbesondere handelt es sich bei dem porösen, keramischen Körper um einen Dentalkeramikrohling, bspw. um einen porös gebrannten keramischen Weißling oder eine porös gepresste Glaskeramik. Ein solcher Dentalkeramikrohling kann als Ausgangskörper für ein erfindungsgemäßes Verfahren dienen. In einem ersten Schritt, der ein Beladungsschritt ist, wird der poröse, keramische Körper mit einer oder mit mehreren Lösungen des einen oder der verschiedenen chemischen Stoffe beladen. Eine Beladung ist im Allgemeinen als das Zuführen der Farbpigmente zu dem porösen, keramischen Körper, vorzugsweise zu einer Oberfläche des porösen, keramischen Körpers zu verstehen. Die Beladung kann insbesondere durch das Auftragen der chemischen Stoffe, die vorzugsweise in einer Lösung enthalten sind, mit einem Pinsel oder einem ähnlichen, geeigneten Applikationswerkzeug, aber auch mittels Besprühen des porösen, keramischen Körpers oder durch simples Eintauchen des porösen, keramischen Körpers in eine Lösung der chemischen Stoffe erfolgen. Um den porösen, keramischen Körper mit den chemischen Stoffen, z. B. Yttriumoxid, Ceroxid oder mit beliebigen anderen organischen und/oder anorganische Reinstoffen und Stoffgemischen, die sich zur Beeinflussung der physikalischen Eigenschaften des keramischen Körpers eignen, zu beladen und diese gezielt innerhalb des porösen, keramischen Körpers zu verteilen sind die chemischen Stoffe in einer flüssigen, insbesondere wässrigen Lösung enthalten. In einem zweiten Schritt, der ein Verteilungssteuerungsschritt ist, wird die Verteilung des einen oder der verschiedenen chemischen Stoffe innerhalb des porösen, keramischen Körpers gesteuert. D.h., die Bewegung der chemischen Stoffe wird derart gesteuert, dass diese an beliebig wählbare Positionen innerhalb des porösen, keramischen Körpers transportiert werden, um einen flächigen bzw. zweidimensionalen Verlauf und/oder einen räumlichen bzw. dreidimensionalen Verlauf der Ausprägungsgrade der einen oder der unterschiedlichen physikalischen Eigenschaften einzustellen. Hierzu werden einer oder mehrere Umgebungsparameter, insbesondere die Luftfeuchtigkeit und/oder der Druck und/oder die Temperatur in einer Umgebung, die ein geschlossenes System bildet, z.B. in einem abgeschlossenen Gefäß, Schrank, Raum oder ähnlichem, in der sich der poröse, keramische Körper befindet, geregelt. Unter einer Regelung wird nicht die ausschließlich eine quantitative Regelung sondern auch eine Regelung bezüglich festgelegter, örtlicher Bereiche innerhalb der Umgebung verstanden, d. h. die poröse Keramik kann bezüglich unterschiedlicher Oberflächen und/oder Oberflächenbereiche mit einem oder mehreren Umgebungsparametern beaufschlagt werden.

In einer vorteilhaften Verfahrensvariante wird die Verteilung des einen oder der verschiedenen chemischen Stoffe innerhalb des porösen, keramischen Körpers durch eine Konvektionsströmung bewirkt. Hierbei wird eine Strömungsrichtung und -geschwindigkeit, vorzugsweise der in Lösung befindlichen chemischen Stoffe, durch gezieltes Erzeugen von Umgebungsparametergradienten innerhalb der Umgebung gesteuert. Insbesondere werden Luftfeuchtigkeitsunterschiede und/oder Druckunterschiede und/oder Temperaturunterschiede bezüglich verschiedener Oberflächen und/oder bezüglich verschiedener Oberflächenbereiche der porösen Keramik eingestellt.

Gemäß einer vorteilhaften Ausführung des Verfahrens wird eine Bewegungsgeschwindigkeit des einen oder der verschiedenen chemischen Stoffe und/oder die Strömungsgeschwindigkeit, insbesondere der in Lösung befindlichen chemischen Stoffe, durch Erhöhen und/oder Verringern eines oder mehrerer der Umgebungsparametergradienten gesteuert. Beispielsweise kann ein erster Umgebungsdruck, der an einer ersten Oberfläche der porösen Keramik anliegt erhöht werden, und ein zweiter Umgebungsdruck, der an einer, der ersten Oberfläche gegenüberliegenden, zweiten Oberfläche der porösen Keramik anliegt verrringert oder konstant gehalten werden, wodurch der Betrag des Druckgradients bezüglich der beiden Oberflächen erhöht wird. Dies führt wiederum zu einer Änderung, insbesondere einer Erhöhung der Bewegungsgeschwindigkeit und/oder der Strömungsgeschwindigkeit.

Gemäß einer ebenso vorteilhaften Ausführung des Verfahrens wird eine Bewegungsrichtung des einen oder der verschiedenen chemischen Stoffe und/oder die Strömungsrichtung, insbesondere der in Lösung befindlichen chemischen Stoffe, durch Änderung der Richtung eines oder mehrerer der Umgebungsparametergradienten gesteuert. Beispielsweise kann der erste Umgebungsdruck an einer ersten Oberfläche des porösen, keramischen Körpers angelegt werden und der zweite Umgebungsdruck an einer anderen, einer dritten Oberfläche des porösen, keramischen Körpers, wodurch die Bewegungs- und/oder eine Strömungsrichtung zwischen der ersten und der dritten Oberfläche des porösen, keramischen Körpers verlaufend gesteuert wird. Durch eine Umkehr des Umgebungsparameter-Gradientenverlaufs (d. h. einer Änderung des Vorzeichens des Gradienten) kann die Bewegungsrichtung und/oder die Strömungsrichtung bspw. zwischen der ersten und der zweiten Oberfläche oder zwischen der ersten und der dritten Oberfläche umgekehrt werden.

In einer optionalen Verfahrensvariante wird mindestens eine Oberfläche oder mindestens ein Teil einer Oberfläche des porösen, keramischen Körpers während des Beladungsschritts und/oder während des Verteilungssteuerungsschritts gegenüber der Umgebung isoliert und/oder abgedichtet. Mindestens eine andere Oberfläche oder mindestens ein anderer Teil einer Oberfläche des porösen, keramischen Körpers ist zur Beladung und/oder zur Steuerung frei zugänglich, d. h. steht mit der Umgebung in Verbindung. Auf diese Weise können die Umgebungsparameter bezüglich festgelegter Oberflächen und/oder Oberflächenteilbereiche örtlich geregelt werden. Die Isolierung und/oder Abdichtung kann während des Beladungsschritts zur örtlich gezielten Beladung des porösen, keramischen Körpers mit dem einen oder den verschiedenen chemischen Stoffe und/oder den in Lösung befindlichen chemischen Stoffen und während des Verteilungssteuerungsschritts zur gezielten Steuerung der Verteilung, insbesondere zur gezielten Steuerung der Bewegungsrichtung und/oder der Strömungsrichtung verwendet werden.

Vorteilhafterweise erfolgt die Beladung des porösen, keramischen Körpers mit dem einen oder den verschiedenen chemischen Stoffe und/oder den in Lösung befindlichen chemischen Stoffen über die mindestens eine, frei zugängliche Oberfläche.

Nach einer optionalen Verfahrensvariante wird die frei zugängliche Oberfläche des porösen, keramischen Körpers vor der Beladung mit dem einen oder den verschiedenen chemischen Stoffe und/oder den in Lösung befindlichen chemischen Stoffen teilweise isoliert und/oder abgedichtet, sodass Teile dieser Oberfläche bzw. Oberflächenbereiche für die Beladung mit dem einen oder den verschiedenen chemischen Stoffe und/oder den in Lösung befindlichen chemischen Stoffen und/oder für die Beaufschlagung mit Umgebungsparametern unzugänglich sind. Vorteilhafterweise wird die frei zugängliche Oberfläche zur Beladung mit dem einen oder den verschiedenen chemischen Stoffe und/oder den in Lösung befindlichen chemischen Stoffen nach unten, in Richtung der Schwerkraft gerichtet.

Optional kann die mindestens eine, frei zugängliche Oberfläche des porösen, keramischen Körpers, während des Verteilungssteuerungsschritts, mit der Umgebung in Verbindung stehen. Die frei zugängliche Oberfläche kann auf diese Weise mit einem oder mehreren der Umgebungsparameter beaufschlagt werden. Ebenfalls während des Verteilungssteuerungsschritts kann die mindestens eine isolierte und/oder abgedichtete Oberfläche gegen einen oder mehrere der Umgebungsparameter abgedichtet und/oder isoliert werden. Auf diese Weise können einzelne Bereiche des porösen, keramischen Körpers gezielt mit Umgebungsparametern beaufschlagt werden, sodass die Richtung und der Ausprägungsgrad der Umgebungsparametergradienten gezielt eingestellt werden, um die räumliche Verteilung des einen oder der verschiedenen chemischen Stoffe innerhalb des porösen, keramischen Körpers zu steuern. Vorteilhafterweise erfolgt eine Abdichtung und/oder Isolierung mittels einer Form, eines Gehäuses oder Ähnlichem und/oder einer Folie und/oder einer Beschichtung. Beispielsweise kann es sich bei der Form um eine Silikonform, bei der Folie um eine selbstklebende Folie und bei der Beschichtung um eine Silikon-, Latex- und/oder Wachsbeschichtung handeln.

Bei einer beispielhaften Verfahrensvariante wird der poröse, keramische Körper zur Abdichtung und/oder Isolierung passgenau in eine Form, insbesondere eine teilgeöffnete Silikonform eingebracht, wobei der Druck innerhalb der Form geringer ist, als bspw. der Umgebungsdruck. In einem Ausführungsbeispiel des Verfahrens ist mindestens eine erste Oberfläche und/oder ein erster Oberflächenteilbereich des porösen, keramischen Körpers innerhalb der Form angeordnet, wobei diese erste Oberfläche und/oder dieser erste Oberflächenteilbereich zumindest gegenüber einem Umgebungsparameter, bspw. gegenüber der Luftfeuchtigkeit, oder dem Druck, die bzw. der in der Umgebung vorliegt, isoliert und/oder abgedichtet ist. Mindestens eine zweite Oberfläche und/oder ein zweiter Oberflächenteilbereich ist außerhalb der Form angeordnet, wobei diese zweite Oberfläche und/oder dieser zweite Oberflächenteilbereich bezüglich der Umgebungsparameter frei zugänglich ist. Mindestens eine dritte Oberfläche und/oder ein dritter Oberflächenteilbereich ist innerhalb der Form angeordnet und liegt passgenau an einer Innenwandung der Form an, sodass innerhalb der Form bspw. ein Unter- oder Überdruck gegenüber dem Umgebungsdruck einstellbar ist. Beispielsweise kann ein Druckunterschied zwischen der innenliegenden, ersten Oberfläche und der außenliegenden, zweiten Oberfläche des porösen, keramischen Körpers eingestellt werden, indem der Druck innerhalb der Form geringer ist als der Umgebungsdruck. Zur Umkehr der Richtung des Druckgradienten kann der Druck innerhalb der Form erhöht oder alternativ der Umgebungsdruck erniedrigt werden, bis der Druck innerhalb der Form höher ist als der an der zweiten, frei zugänglichen Oberfläche anliegende Umgebungsdruck. Alternativ kann der poröse, keramische Körper zur Umkehr der Richtung des Druckgradienten innerhalb der Form gewendet werden, sodass die erste Oberfläche frei zugänglich außerhalb der Form angeordnet ist und die zweite Oberfläche isoliert und/oder abgedichtet innerhalb der Form angeordnet ist. Optional kann der poröse, keramische Körper mit zusätzlichen Umgebungsparametergradienten, wie beispielsweise Luftfeuchtigkeitsunterschieden beaufschlagt werden, wobei die Luftfeuchtigkeit vorzugsweise an der zweiten, frei zugänglichen Fläche geregelt wird. Durch die Verwendung von selbstklebender Folie auf der zweiten, frei zugänglichen Oberfläche kann ein Umgebungsparameter, wie die Luftfeuchtigkeit bezüglich einzelner, zweiter Oberflächenteilbereiche geregelt werden.

Optional kann die Beladung des porösen, keramischen Körpers mit dem einen oder den verschiedenen chemischen Stoffen und/oder den in Lösung befindlichen chemischen Stoffen mittels eines Beladungskörpers erfolgen. Insbesondere umfasst der Beladungskörper ein poröses und/oder schwammartiges Material, das die Aufnahme des einen oder der verschiedenen chemischen Stoffe und/oder der in Lösung befindlichen chemischen Stoffe begünstigt. Der Beladungskörper ist hierbei mit einem Lösungsmittel und darin enthaltenen chemischen Stoffen versetzt, insbesondere gesättigt. Vorzugsweise wird der poröse, keramische Körper zur Beladung mit dem einen oder den verschiedenen chemischen Stoffe und/oder den in Lösung befindlichen chemischen Stoffen während des Beladungsschritts mit einer frei zugänglichen Oberfläche auf den Beladungskörper aufgelegt. Die optionale Beladungsmethode bietet den Vorteil, dass die gesamte, frei zugängliche Oberfläche des porösen, keramischen Körpers mit dem Beladungskörper in berührender Verbindung steht, wodurch diese gleichmäßig mit den chemischen Stoffen beaufschlagt wird. Die Konzentration des einen oder der verschiedenen chemischen Stoffe, die dem porösen, keramischen Körper während des Beladungsschritts zugeführt werden, lässt sich auf diese Weise über die gesamte, frei zugängliche Fläche konstant halten.

Der Beladungskörper kann beispielsweise eine oder mehrere Schichten umfassen, wobei die eine oder die mehreren Schichten denselben chemischen Stoff zur Einstellung eines Verlaufs und/oder eines räumlichen Verlaufs des Ausprägungsgrads einer einzelnen physikalischen Eigenschaft innerhalb des porösen, keramischen Körpers enthalten. Um einen Verlauf und/oder einen räumlichen Verlauf der Ausprägungsgrade unterschiedlicher physikalischer Eigenschaften innerhalb des porösen, keramischen Körpers einzustellen, können verschiedene Schichten mit verschiedenen chemischen Stoffen bzw. deren Lösungen versetzt und/oder gesättigt werden. Die mehreren Schichten des Beladungskörpers können horizontal nebeneinander und/oder vertikal untereinander bzw. übereinander angeordnet sein. Mittels vertikal angeordneter Schichten, wird bereits bei der Beladung ein vertikaler Verlauf des Ausprägungsgrads innerhalb des porösen, keramischen Körpers begünstigt oder erzeugt. Der zu beladende, poröse keramische Körper steht lediglich mit der obersten Schicht des Beladungskörpers in Verbindung bzw. liegt auf der obersten Schicht des Beladungskörpers auf. Die chemischen Stoffe bzw. deren Lösungen, die in den übrigen Schichten enthalten sind, durchlaufen die jeweils oberhalb angeordneten Schichten, bevor sie in den porösen, keramischen Körper eindringen. Auf diese Weise lässt sich eine zeitlich versetzte Beladung mit verschiedenen chemischen Stoffen, die in einem Verlauf der Ausprägungsgrade resultiert, erzielen. Eine horizontale Anordnung der Schichten, kann bereits während der Beladung des porösen, keramischen Körpers einen horizontalen Verlauf der Ausprägungsgrade innerhalb des porösen, keramischen Körpers begünstigen oder erzeugen. Der zu beladende, poröse, keramische Körper steht hierbei mit allen horizontal nebeneinander angeordneten Schichten in Verbindung bzw. liegt auf diesen auf, sodass die chemischen Stoffe der einzelnen Schichten gleichzeitig, jedoch an verschiedenen Oberflächenteilbereichen der frei zugänglichen Oberfläche in den porösen, keramischen Körper eindringen. Eine Kombination von vertikal über- bzw. untereinander und horizontal nebeneinander angeordneten Schichten begünstigt oder erzeugt bereits bei der Beladung einen dreidimensionalen bzw. räumlichen Verlauf der Ausprägungsgrade einer oder verschiedener physikalischer Eigenschaften innerhalb des porösen, keramischen Körpers. Je nach gewünschtem Endergebnis kann eine Kombination von Schichten horizontal nebeneinander und vertikal über- bzw. untereinander beliebig erfolgen. Natürlich können verschiedene chemische Stoffe zur Herstellung eines keramischen Körpers mit Ausprägungsgradverläufen unterschiedlicher physikalischer Eigenschaften auch nacheinander, in mehreren Beladungschritten, mit jeweils einer einzigen Schicht des Beladungskörpers dem porösen, keramischen Körper zugeführt werden. Diese Methode ist besonders vorteilhaft in Verbindung mit einer 3D-gedruckten Keramik-Struktur. Im Gegensatz zu einem gepressten Keramikrohling weist die gedruckte Keramik-Struktur eine wesentlich höhere Porosität bzw. eine wesentlich geringere Dichte auf, wodurch die Weiterverarbeitung bspw. mittels einer Keramikfräse erschwert ist. Durch mehrere, aufeinanderfolgende Beladungsschritte lässt sich die Dichte einer gedruckten Keramik-Struktur bspw. von 0,8 g/mm³ auf 3,5 g/mm³ erhöhen, indem die Poren bzw. die Porosität der Keramik-Struktur mit den chemischen Stoffen befüllt werden.

Nach einer optionalen Verfahrensvariante umfasst der Beladungskörper einen Filter. Der Filter bildet vorzugsweise die oberste Schicht des Beladungskörpers und kann mit einem Lösungsmittel, ohne Zusatz chemischer Stoffe, die zur Beeinflussung physikalischer Eigenschaften geeignet sind, versetzt bzw. gesättigt sein. Durch die Verwendung des Filters, der insbesondere ein gleiches oder ähnliches Material wie die übrigen Schichten des Beladungskörpers umfasst, kann eine gleichmäßigere Verteilung der chemischen Stoffe während des Beladungsschritts und somit eine einfachere Steuerung des Verlaufs der Ausprägungsgrade erzielt werden. Insbesondere enthält der Filter ein mit Zirkon-Nitrat versetztes Lösungsmittel.

Vorteilhafterweise erfolgt eine Trocknung des porösen, keramischen Körpers, insbesondere eine vollständige Trocknung bereits während des Verteilungssteuerungsschritts. Beispielsweise können die Umgebungsparameter, insbesondere die Luftfeuchtigkeit und/oder der Druck und/oder die Temperatur zur Steuerung des Trocknungsvorgangs geregelt werden, wodurch einerseits die Trocknungszeit, andererseits der örtliche Trocknungsverlauf gezielt variiert werden kann. So kann eine Trocknung insbesondere von einer, mehreren oder sämtlichen frei zugänglichen Oberflächen des porösen, keramischen Körpers aus erfolgen. Insbesondere lassen sich die chemischen Stoffe durch Trocknung, d.h. Verdunstung des Lösungsmittels ortsfest innerhalb des porösen, keramischen Körpers an der gewünschten Position fixieren.

Gemäß einem beispielhaften, erfindungsgemäßen Verfahrensablauf wird in einem ersten Schritt ein, z. B. flacher und/oder plattenförmiger, poröser Keramikrohling und/oder ein Keramikgerüst bzw. eine poröse Keramikstruktur, insbesondere ein Weißling, der zur Verwendung im Dentalbereich geeignet ist, bereitgestellt. Plattenförmige Dental-Keramikrohlinge sind im Handel erhältlich und eignen sich zur Verarbeitung mit einer herkömmlichen CAD/CAM-Keramikfräsmaschine und zur anschließenden Endsinterung, um einen fertigen Zahnersatz herzustellen. In einem zweiten Verfahrensschritt werden eine oder mehrere Oberflächen des Keramikrohlings mit einer Isolierung und/oder Abdichtung versehen, wobei der Keramikrohling in einer wasser- und luftundurchlässigen, d.h. dichtenden Form, insbesondere einer Silikonform platziert wird. Hierbei wird mindestens eine Oberfläche oder ein Oberflächenteilbereich des Keramikrohlings nicht von der Form isoliert und/oder abgedichtet, sodass diese Oberfläche zur Beaufschlagung mit Umgebungsparametern bzw. zur Beladung frei zugänglich ist. In einem dritten Schritt, einem Beladungsschritt, wird die frei zugängliche Oberfläche bzw. der frei zugängliche Oberflächenbereich des Keramikrohlings mit einem oder verschiedenen chemischen Stoffen, die zur Beeinflussung der physikalischen Eigenschaften des Keramikrohlings geeignet sind beladen, wobei die chemischen Stoffe, bspw. Cer, Yttrium, Calcium und/oder Magnesium, in einer flüssigen, insbesondere wässrigen Lösung enthalten sind bzw. in Lösung vorliegen. Der Keramikrohling wird in einem vierten Schritt innerhalb einer Umgebung, deren Umgebungsparameter, insbesondere die Luftfeuchtigkeit und/oder der Druck und/oder die Temperatur regelbar sind, platziert. Hierbei kann es sich beispielsweise um einen Klimaschrank oder Trockenschrank, aber auch einen begehbaren Raum, dessen Umgebungsparameter regelbar sind, handeln. Der Keramikrohling kann hierbei in der Form belassen werden, sodass lediglich die frei zugängliche Oberfläche mit der Umgebung in Verbindung steht. In einem fünften Schritt, einem Verteilungssteuerungsschritt, wird die Verteilung der chemischen Stoffe, die mittels der Beladung in den Keramikrohling eingebracht wurden, innerhalb des Keramikrohlings gesteuert. Hierzu wird mindestens ein Umgebungsparameter, insbesondere die Luftfeuchtigkeit und/oder der Druck und/oder die Temperatur zur Erzeugung eines Umgebungsparametergradienten zwischen der einen oder den mehreren frei zugänglichen Oberflächen und der einen oder den mehreren isolierten und/oder abgedichteten Oberflächen des Keramikrohlings geregelt.

Alternativ oder optional kann der poröse, keramische Körper bzw. der Keramikrohling in einem zusätzlichen Trocknungsschritt wärmebehandelt werden, der sich an den Verteilungssteuerungsschritt bzw. an die Verteilung der chemischen Stoffe innerhalb des porösen, keramischen Körpers anschließt. Der poröse, keramische Körper oder der Keramikrohling wird hierbei zur Ausbildung einer Oxidphase, insbesondere einer Nitratoxidphase einer Temperatur in einem Bereich zwischen 80°C und 1200°C, vorzugsweise zwischen 80°C und 800°C ausgesetzt. Mit Sauerstoff können in Lösung befindliche Kationen, bspw. von Salzen, die als chemische Stoffe zur Beeinflussung der physikalischen Eigenschaften dem Lösungsmittel beigesetzt sind, Oxide bzw. eine Oxidphase bilden. Insbesondere wird der poröse, keramische Körper einer Wärmebehandlung unterzogen, um einerseits eine örtliche Fixierung und andererseits eine Entwicklung der physikalischen Eigenschaften zu begünstigen. Durch die Zugabe von Zirkon-Nitrat zu dem Lösungsmittel bzw. zu den in Lösung befindlichen, chemischen Stoffen sowie einen Trocknungsschritt mit Wärmebehandlung lassen sich die chemischen Stoffe zur Einstellung physikalischer Eigenschaften des porösen, keramischen Körpers zuverlässig an der gewünschten Position fixieren. Aufgrund der Wärmebehandlung bildet das Zirkon-Nitrat eine feste Struktur innerhalb der Poren der porösen Keramik, welche die chemischen Stoffe einschließt bzw. fixiert. Die Wärmebehandlung ermöglicht insbesondere ein Fräsen mit wasserkühlenden CAD/CAM-Maschinen, ohne dass eine Delokalisierung bzw. eine Verschiebung der chemischen Stoffe innerhalb der porösen Keramik zu befürchten ist. Je nach verwendeter Keramik werden Temperaturen von bspw. 700°C - 2000°C, in einer Luft- oder Inertgasatmosphäre (z. B. Stickstoff, Argon...) bei Umgebungsdruck oder in einem Vakuum eingestellt und die Keramik diesen Bedingungen ausgesetzt, bis eine Dichte von ca. 94% - ca. 100% der endgültigen, d.h. der nach dem Endsintern vorliegenden Dichte, erreicht ist.

Während und/oder nach der Verteilung der chemischen Stoffe innerhalb des porösen, keramischen Körpers kann gemäß einer optionalen Verfahrensvariante ein WAK-Ausgleich durchgeführt werden, wobei der poröse, keramische Körper zumindest teilweise mit einem Ausgleichsstoff beladen wird. Zur Fertigstellung eines Zahnersatzes wird der Keramikrohling üblicherweise einem Endsintervorgang, d.h. einer Hochtemperaturbehandlung unterzogen, wodurch eine Werkstoffverdichtung erzielt wird und die Porenräume aufgefüllt werden. Aufgrund unterschiedlicher Wärmeausdehnungskoeffizienten (WAK) der verschiedenen Materialien, bspw. des Keramikrohlings sowie der infiltrierten chemischen Stoffe können Spannungen durch voneinander abweichende, wärmebedingte Volumenausdehnungen auftreten. Derlei Spannungen führen häufig zu einer Rissbildung, wodurch der keramische Körper als Zahnersatz unbrauchbar wird. Durch Zugabe eines Ausgleichsstoffs werden die unterschiedlichen WAK-Werte angeglichen und Spannungen sowie Rissbildungen vermieden.

In einem optionalen, zusätzlichen Verfahrensschritt kann der poröse, keramische Körper oder der Keramikrohling nach der Verteilung und gegebenenfalls Fixierung der chemischen Stoffe mittels einer CAD/CAM-Fräsmaschine in die gewünschte Form, insbesondere in die Form eines Zahnersatzteils gefräst werden. In einem weiteren optional zusätzlichen Verfahrensschritt kann der poröse, keramische Körper oder der Keramikrohling nach der Verteilung und gegebenenfalls Fixierung der chemischen Stoffe, sowie vorzugsweise nach einer formgebenden Bearbeitung wie beispielsweise Fräsen, gesintert bzw. endgesintert werden. Hierdurch werden die Poren geschlossen, womit die chemischen Stoffe fixiert sind, sodass sich ein der Verteilung entsprechender räumlicher Verlauf einer oder mehrerer verschiedener physikalischer Eigenschaften ergibt. Insbesondere kann der Keramikrohling vor der Verteilung der chemischen Stoffe bei niedrigerer Temperatur angesintert und nach der Verteilung der chemischen Stoffe bei höherer Temperatur endgesintert werden.

Gemäß einer optionalen Verfahrensvariante werden die eine oder die mehreren Lösungen eines oder verschiedener chemischer Stoffe derart innerhalb des porösen, keramischen Körpers gesteuert, dass sich Konzentrationsunterschiede der gelösten chemischen Stoffe innerhalb der verschiedenen Bereiche des porösen, keramischen Körpers einstellen. Die unterschiedliche Konzentration führt zu einem unterschiedlichen Ausprägungsgrad der physikalischen Eigenschaften. Bereits durch die Einstellung unterschiedlicher Konzentrationen der chemischen Stoffe, die in der Lösung enthalten sind, kann die Dichte des porösen, keramischen Körpers räumlich gezielt eingestellt werden. Nach einer Trocknung bzw. einer Verdunstung des Lösungsmittels verbleiben die chemischen Stoffe innerhalb der Poren und führen ortsspezifisch zu einer Erhöhung der Dichte.

Vorzugsweise enthält die Lösung, mit der der poröse, keramische Körper beladen wird bzw. deren Verteilung innerhalb des porösen, keramischen Körpers gesteuert wird, destilliertes Wasser, Zirkon-Nitrat sowie mindestens einen chemischen Stoff, der zur Beeinflussung der physikalischen Eigenschaften des porösen, keramischen Körpers geeignet ist. Insbesondere kann ein Ausprägungsgrad einer Opazität und/oder einer Transluzenz des porösen, keramischen Körpers mittels einer Yttrium enthaltenden Lösung gesteuert werden. Das Yttrium wird vorzugsweise als Yttrium-Nitrat oder Yttrium-Chlorid zugegeben. Ein Ausprägungsgrad einer Härte und/oder einer Zähigkeit und/oder einer Festigkeit des porösen keramischen Körpers kann insbesondere mittels einer Cer enthaltenden Lösung gesteuert werden. Vorzugsweise wird das Cer in Form von Cer-Nitrat oder CerChlorid der Lösung zugesetzt.

Nach einer vorteilhaften Verfahrensvariante kann eine Konfiguration eines Kristallsystems des porösen, keramischen Körpers oder einzelne Bereiche des porösen, keramischen Körpers mittels einer Calcium und/oder Magnesium und/oder Yttrium enthaltenden Lösung gesteuert werden. Um das Kristallsystem des porösen, keramischen Körpers zumindest bereichsweise in einer kubischen Konfiguration zu stabilisieren kann der Lösung ein Molanteil von mindestens 16 % Calcium und/oder 16 % Magnesium und oder 8 % Yttrium zugesetzt sein. Zur Stabilisierung des Kristallsystems des porösen, keramischen Körpers zumindest bereichsweise in einer tetragonalen Konfiguration kann der Lösung ein Molanteil von mindestens 8 % Calcium und/oder 8 % Magnesium und/oder 4 % Yttrium zugesetzt sein. Die kristalline Konfiguration des porösen, keramischen Körpers nimmt wesentlichen Einfluss auf dessen physikalische Eigenschaften womit die physikalischen Eigenschaften des porösen, keramischen Körpers, mittelbar durch die Steuerung der Verteilung chemischer Stoffe, die zur Beeinflussung der Konfiguration des Kristallsystems geeignet sind, eingestellt werden.

In einer optionalen, vorteilhaften Verfahrensvariante wird ein Kristallsystem innerhalb von Poren des porösen, keramischen Körpers mittels einer, insbesondere wässrigen Lösung, die Zirkon-Nitrat sowie Calcium und/oder Magnesium und/oder Yttrium umfasst, stabilisiert. Je nach Molanteil des Calciums und/oder des Magnesiums und/oder des Yttriums (s. o.) wird das Kristallsystem in einer kubischen bzw. einer tetragonalen Konfiguration stabilisiert. Vorzugsweise wird der poröse, keramische Körper derart mit der Lösung beladen bzw. wird die Verteilung der Lösung derart innerhalb der Poren des porösen, keramischen Körpers gesteuert, dass die Poren vollständig oder bereichsweise mit einem Kristallsystem in tetragonaler und/oder kubischer Konfiguration füllbar sind.

Das zuvor beschriebene, erfinderische Verfahren lässt sich zusätzlich oder alternativ, durch Beladung mit zusätzlichen Farbpigmenten, beispielsweise in Form von Metalloxiden, zur monochromen, polychromen oder räumlich polychromen Einfärbung eines porösen, keramischen Körpers verwenden. Der poröse, keramische Körper wird zusätzlich zu den in Lösung befindlichen chemischen Stoffen mit einer oder mehreren unterschiedlichen Farbpigmentlösungen, insbesondere Metalloxidlösungen, beladen. Anschließend wird deren Verteilung innerhalb des porösen, keramischen Körpers gesteuert. Optional können die Farbpigmente auch direkt den Lösungen der chemischen Stoffe, die zur Beeinflussung der physikalischen Eigenschaften des porösen, keramischen Körpers geeignet sind, zugegeben werden.

Die Erfindungsaufgabe wird ferner durch einen Keramikrohling, der zur Herstellung von Zahnersatz mittels einer CAD/CAM-Fräsmaschine geeignet ist und insbesondere in einem erfindungsgemäßen Verfahren hergestellt wurde, gelöst. Eine räumliche Verteilung eines oder verschiedener chemischer Stoffe, die zur Beeinflussung der physikalischen Eigenschaften des Keramikrohlings geeignet sind, innerhalb des Keramikrohlings ist mittels Umgebungsparametergradienten steuerbar. Nach einer Sinterbehandlung, insbesondere einem Endsintern bei hoher Temperatur oder einer Wärmebehandlung bei niedrigerer Temperatur weist der Keramikrohling einen graduellen und/oder abgestuften, vorzugsweise räumlichen Verlauf des Ausprägungsgrades einer oder mehrerer unterschiedlicher physikalischer Eigenschaften auf.

Der nachfolgenden Tabelle sind beispielhafte Werte bzw. beispielhafte Bereiche der Werte der physikalischen Eigenschaften, die mittels des erfinderischen Verfahrens räumlich gezielt in Bereichen des keramischen Körpers eingestellt werden können, zu entnehmen. Die Werte beziehen sich jeweils auf einen endgesinterten, d. h. dicht gesinterten keramischen Körper:

### Zirkonoxidkeramik

| | | | | | | |
|---|---|---|---|---|---|---|
| **Zusammensetzung** | **%** | **70 bis** | **99 ZrCO₂** | | | |
| **Dichte** | **g/cm³** | **5,0 bis** | **6,5** | **DIN** | **EN** | **623-2** |
| **offene Porosität** | **%** | **0** | | | | |
| **Korngrösse (mli)** | **µm** | **0,01** | **bis 35** | **DIN** | **EN** | **623-3** |
| **Härte Vikers** | **Hv** | **350 bis** | **1500** | **DIN** | **EN** | **843-4** |
| **Biegefestigkeit (3Punkte)** | **MPa** | **500 bis** | **2500** | **DIN** | **EN** | **843-1** |
| **Elastizitätsmodul** | **GPa** | **150 bis** | **250** | **DIN** | **EN** | **843-2** |
| **Bruchzähigkeit** | **MPa/m²** | **4 bis** | **16** | | | |
| **chemische Lösilchkeit** | **µm /cm²** | **1 bis** | **250** | **EN** | **ISO** | **6872** |
| **Wärmeausdehnung (20-600°C)** | **10°/K** | **8 bis** | **12** | **DIN** | **EN** | **821** |
| **Sinterschwindung** | **%** | **0 bis** | **35** | | | |
| **mögliche Herstellungsverfahren keramischer Rohlinge** | | | | | | |
| **isostatisches Pressen** | **für erfindungsgemäßes Verfahren verwendbar** | | | | | |
| **Formpressen** | **für erfindungsgemäßes Verfahren verwendbar** | | | | | |
| **Schlickerguss** | für **erfindungsgemäßes Verfahren verwendbar** | | | | | |
| **HIP** | **für erfindungsgemäßes Verfahren verwendbar** | | | | | |
| **3D-Druck** | **für erfindungsgemäßes Verfahren verwendbar** | | | | | |

Teil des erfinderischen Gedankens ist ebenfalls eine Vorrichtung zur Beladung eines porösen, keramischen Körpers, insbesondere eines Dental-Keramikrohlings mit einer Lösung chemischer Stoffe, die zur Beeinflussung der physikalischen Eigenschaften des porösen, keramischen Körpers geeignet sind. Die Beladungsvorrichtung umfasst ein poröses und/oder schwammartiges Material, das mit der Lösung versetzbar, vorzugsweise sättigbar ist, sodass die Lösung an den beispielsweise aufliegenden, porösen keramischen Körper abgebbar ist bzw. der poröse, keramische Körper mit dieser beladbar ist. Gemäß einer vorteilhaften Ausführung umfasst die Beladungsvorrichtung mindestens zwei Schichten, wobei mindestens eine Schicht als Filter ausgebildet, d.h. insbesondere nicht mit chemischen Stoffen zur Beeinflussung der physikalischen Eigenschaften versetzt ist und/oder mindestens zwei Schichten mit jeweils verschiedenen chemischen Stoffen zur Beeinflussung unterschiedlicher physikalischer Eigenschaften versetzt sind.

Weiterhin gehört eine Vorrichtung zur Steuerung einer gezielten Verteilung von chemischen Stoffen innerhalb eines porösen, keramischen Körpers, insbesondere eines Dental-Keramikrohlings zum allgemein erfinderischen Gedanken. Die chemischen Stoffe eignen sich zur Beeinflussung physikalischer Eigenschaften des porösen, keramischen Körpers. Die Steuerungsvorrichtung ist zur Isolierung und/oder Abdichtung von mindestens einer Oberfläche und/oder mindestens eines Teils einer Oberfläche des porösen, keramischen Körpers geeignet, wobei mindestens eine andere Oberfläche und/oder mindestens ein anderer Teil einer Oberfläche zur Beaufschlagung mit regelbaren Umgebungsparametern frei zugänglich ist. Beispielsweise handelt es sich bei der Vorrichtung um eine geöffnete Form, insbesondere eine Silikonform, die zur passgenauen Aufnahme des porösen, keramischen Körpers ausgebildet ist, sodass der poröse, keramische Körper formschlüssig von der Silikonform aufgenommen wird, wobei eine Oberfläche des porösen, keramischen Körpers frei zugänglich ist.

Den nachfolgenden Abschnitten sind weitere beispielhafte Merkmale, Merkmalskombinationen und Ausgestaltungen im Rahmen der Erfindung zu entnehmen.

Das erfindungsgemäße Verfahren zum Färben und/oder Einstellen physikalischer Eigenschaften, insbesondere zur Herstellung eines monochromen, polychromen oder räumlich polychromen Dentalrohlings mit physikalisch einstellbaren Zonen in durchgängiger Keramik, benötigt keine Pulvermischungen oder Pasten, aufwendige Schichtungen/Schüttungen und/oder Wechselbecken der 3D-Drucker und/oder Spezialwerkzeuge. Das Verfahren beruht auf Verteilungssteuerungsschritten die am besten mit der bekannten Konvektionsströmung erklärbar ist, durch Beladung von komplexen Pigmenten und/oder stabilisatorischen Lösungen in die Porositäten, eines vorproduziertem keramischen Rohlings, insbesondere für die Dentaltechnik , sowie einem der Erzeugung von Umgebungsparameter Gradienten anzugeben. Bei dem die aus dem Stand der Technik geschilderte Nachteile vermieden und auch ein verkaufsfertiger Keramikrohling aus dem Lagerbestand wunschgemäß eingefärbt wird und räumlich vorteilhafte Zonen durch Beladung weiterer Zirkonstabilisatoren Kombinationen dotiert und eingestellt werden. Die Lehre der US 2015 028 2905 oder US 2011 269 618 lehren aufwendige Pulver Herstellungsverfahren um ein verbessertes Zirkonpulver herzustellen. Diese Lehren führen zu einer Erhöhung der Pulverkosten. Erfindungsgemäß ist es erstaunlicher Weise möglich mit den Eigenschaften von einfach hergestellten Pulvern einen porösen keramischen Rohling herzustellen, die keinen hohen Kostenfaktor aufweisen und daher größere Porositäten haben. Diese werden in einem Beladungs- und Verteilungssteuerungsschritt mit einer Salzlösung beladen, die zu einer Kristallbildung in den Porositäten führt, durch eine Wärmebehandlung entsteht eine Kristalloxidphasenumwandlung. Die Oxidation führt dann zu Oxiden die sich in den entsprechenden Porositäten und deren Platzverhältnissen anpassen. So kommt es zu einer Weißlingsdichte die durch eine reine Formpressung und/oder isostatisches und/oder Hip Pressungsverfahren nicht erreichbar sind. Die Weißlingsdichte kann somit doppelt so hoch sein und aufwendige Pulverherstellungsverfahren oder deren aufwendigen Schichtungen und Pressverfahren zu verwenden. Was somit auch zu den erfindungsgemäßen physikalisch einstellbaren Zonen führt, was gerade wichtig bei den porösen Keramik-Strukturen des Keramik-3D-Druckverfahrens ist. Somit sind erfinderische Verfahren zur Herstellung eines polychromen und/oder räumlich polychromen und/oder eines monochromen gefärbten Dentalrohling mit physikalischen einstellbaren Zonen, dadurch gekennzeichnet das in einem ersten keramischen Gerüst mit kubisch Zirconia oder tetragonal oder monoklin teil- oder vollstabilisiertes Oxid vorliegt und das in einem zweiten Schritt ein weiteres kubisches oder tetragonal und/der monolithisches teil- oder vollstabilisiertes dotiertes Zirkonoxid in den Porositäten entsteht und somit mindestens ein Kristallgitter als ein zweites in das Zirkonoxidgitter eingebracht wird, das homogen oder hochkonzentriert verteilt werden kann. Sodas die Rissfähigkeit erhöht und/oder die Härte gesengt werden kann. Dies trägt auch zu einer Kostenreduzierung in der Zahnarztpraxis bei, da ein weicher Werkstoff sehr viel schneller eingeschliffen werden kann (teure Einpassarbeiten im Mund beim Zahnarzt).

Die Einstellbarkeit räumlicher Zonen bzw. Bereiche mit unterschiedlichen physikalischen Eigenschaften und oder unterschiedlichen Ausprägungsgraden physikalischer Eigenschaften ist vorteilhaft, da eine hohe Vickershärte, Biegefestigkeit und Bruchzähigkeit in einem einphasigen Körper nicht räumlich zu trennen sind. Erfindungsgemäß lassen sich aber erstaunlicher Weise mehrere Phasen unterschiedlicher physikalischer Eigenschaften in einen keramischen Körper einbringen. Das hat den Vorteil, dass eine kostenaufwendige Bearbeitung der endgesinterten Zirkonkeramik um 30-50% gesenkt werden kann. Das endgesinterte Zirkonoxid ist eine Hochleistungskeramik mit einer Biegefestigkeit von ca.1100 MPa. Dentalwerkstoffe aus denen Brücken hergestellt werden, müssen eine Biegefestigkeit von min. 500 MPa haben. ZrO2 (Zirkoniumoxid) gehört zur Gruppe der Oxidkeramiken, ist also ein nichtmetallischer organischer Werkstoff. Es wird auch als "keramischer Stahl" bezeichnet. Die Biegefestigkeit ist mehr als doppelt so hoch wie bei Empress und fast doppelt so hoch wie bei infiltrierten Aluminiumoxidkeramiken (z.B. Inceram Alumina). Mit der Bruchzähigkeit verhält es sich ähnlich. Das Ermüdungsverhalten übertrifft die glasinfiltrierten Keramiken um das 3-fache. Bei Zirkonoxidkeramik handelt es sich in der Regel um mit Yttriumoxid (Zusatz etwa 3 mol-%) teilstabilisiertes tetragonales polykristallines Zirkonoxid (Y-TZP=Yttria-Tetragonal Zirconia Polycristals).Diese Stabilisierung wird als Umwandlungsverstärkung bezeichnet und bewirkt eine gewisse Rissstopfunktion. An der Rissspitze wirkende Zugspannung induziert eine Umwandlung des metastabilen tetragonalen Zirkonoxids in die thermodynamisch günstigere monokline Form. Diese Umwandlung ist mit einem lokalen Volumenzuwachs verbunden. Dadurch wird örtlich begrenzt an der Rissspitze eine Druckspanne erzeugt, die der von außen wirkenden Belastung an der Rissspitze entgegenwirkt. Dies führt zu hohen initialen Festigkeiten und Risszähigkeiten sowie in Kombination mit einer geringen Anfälligkeit gegenüber Spannungskorrosion zur hervorragenden Lebensdauer für Zirkonoxid-Gerüste. Die yttriumstabilisierte Zirkonoxidkeramik setzt sich wie folgt zusammen: Zirkonoxid, Hafniumdioxid und Yttriumoxid zusammen >99.0% Aluminiumoxid und andere Oxide jeweils < 0,5%. Zirkon ist ein Metall der Titangruppe. Zirkonoxid gehört zur Gruppe der Oxidkeramiken. Polykristallines Oxidkeramiken bilden direkt nach dem Sintern eine dichte einphasige Oxidkeramik. Diese Eigenschaften können aber Erfindungsgemäß verbessert und polychromatisch und räumlich physikalisch eingestellt werden. Zirkonoxid wird durch z.B. die chemische Behandlung von Zirkonsand (chemische Zusammensetzung ZrSiO4) hergestellt. Nach der chemischen Auflösung und Reinigung entsteht ein hochreiner Grundstoff, der später mit Yttriumoxid(Y2O3)dotiert, wärmebehandelt und schließlich gemahlen wird. Das gewonnene Zirkonoxid ist praktisch frei von allen störenden Verunreinigungen. Es wird Anschließend in Blöcke unterschiedlicher Größe (Rohlinge) gepresst. In diesem sogenannten "Soft "Zustand ist es leicht bearbeitbar. Dieser Weißling ist z.B. Ausgangsrohling des erfinderischen Verfahrens.

Bei dem vorgeschlagenen Verfahren zum Färben und einstellen physikalischer Eigenschaften von gebinderter und/oder entbinderter und/oder angesinterter und/oder durchgängig poröser Keramik, insbesondere von porösen Körpern, die insbesondere in der Dentaltechnik Anwendung finden, erfolgt die farbgebende Verteilung und/oder Stabilisator in Keramikkörper und/oder auch in verkaufsfertigen Keramikrohlingen in einem dichten z.B. Silikongehäuse, um auch die Bewegungsrichtung der Farbpigmente und/oder Stabilisatorenlösungen gesteuert vorzugeben. In dem z.B. Silikongehäuse befindet sich auch ein Raum mit und/oder ohne sphärischen Druck mit und/oder Beladungskörperstoffe .zur luftfreien kontinuierlichen Befüllung der Porositäten. Unter einem Abdichtung- und/oder Isolierungsmittel einer Form versteht man alle Materialien die Flächen eines porösen Keramikrohlings dicht oder luftdurchlässig und/oder umgeben können. Vorzugsweise wird eine Fläche des porösen Keramikrohlings auf einen kapillardruckhaltenden Beladungskörperstoff Lösungsmittelvorrichtung aufgesetzt, in der oder auf der Farbpigmentlösung und/oder Stabilisatorenlösungen gespeichert sind. Vorteilhaft ist die Auflage zum luftfreien Transport aus Beladungskörperstoffen herzustellen, wie Mikrofaser, Schwämme, Zellstoff usw. Unter dem Begriff Beladungskörperstoff versteht man somit alle Materialien oder Stoffe die wasserdurchlässig sind, und/oder diese speichern können. Die Beladungskörperstoffe erfüllen einen wichtigen Zweck. Die Kapillarkraft der porösen, isostatisch gepressten Weißlinge ist so stark, dass z.B. unsere Zunge sofort an der porösen Keramik kleben bleibt. Das liegt an der hohen Dichte der porösen Keramik von 0,5 g/cm³ bis 4,0 g/cm³. Wird die poröse Keramik mit Pinsel oder Flüssigkeiten beauftragt, wird diese sofort weggesaugt, aber die farbgebenden Komponenten, sind langsamer als die Lösungsflüssigkeit. So wirkt die poröse Keramik wie ein Filter, in dem sich die farbgebenden Komponenten dann irgendwo verstopft konzentrieren. Der Verteilungssteuerungsschritt kann diese auflösen und neu im Keramikblock verteilen. Dieses kann aber mehrere Tage in dem z.B. Silikongehäuse mit dem entsprechenden Umgebungsparameter bedeuten. Dem zu Grunde wird je nach Porosität des Keramikrohlings ein Beladungskörperstoff ausgesucht, der flüsssigkeitsbremsend wirkt. Somit kommt es zu wesentlich weniger ungewünschten Konzentrationsansammlungen. Die farbgebenden Komponenten können dann schneller vom Verteilungssteuerungsschritt eine gewünschte farbgebenden Verteilung erreichen. Des Weiteren lassen sich verschiedene farbgebende Komponenten und/oder Stabilisatoren in zugeordneten Volumen in Schichten aufbauen, das heißt 50ml Gesamtvolumen der vorhanden keramischen Porositäten können in jeweils 10ml verschiedene Beladungskörperstoffen eingebracht werden. Durch gleichen Kapillardruck können jetzt fünf verschiedene Farbkomponente Lösungen und/oder Stabilisatorenlösungen übereinander gelegt und gespeichert werden, ohne dass sich diese vermischen, was in einer Flüssigkeit ansonsten nahezu unmöglich ist. Durch die kapillardruckenthaltenden Beladungskörperstoffvorrichtung entsteht so eine Kapillarunterdruckbeladung, weil eine farbgebende Lösung den Druck an die andere weiter gibt und somit verlaufende Farbübergänge entstehen und das auch in gewünschten Zonen des keramischen Rohlings nur mit Stabilisationskomplexen beladen werden mit und ohne Druck. Das bedeutet, dass die Beladungskörperstoffe den gewünschten Kapillardruck bei feuchtem und/oder nassem Untergrund aufnehmen. Z.B der farbgebenden Komponenten für Salze sind: Sinitnitrat - Nanohytrate, Oxidhydrate - Nitrate, Tetranitrate-Nitrate, Pentahytrate - Nitrate Hexa, Chloride, Azetate, Niobate, Metavanadate oder Sulfide usw. In der Regel lassen sich die entsprechenden Lösungen in einfacher Weise dadurch herstellen, dass ein entsprechendes Z. B. Metallsalz in den entsprechenden Lösungsmitteln, vorzugsweise Wasser aufgelöst wird. Vorzugsweise wird bei der Erfindung dabei von den entsprechenden Salzen wie Chloraten, Sulfaten, Carbonaten oder insbesondere Nitraten des z.B. jeweiligen Metalls ausgegangen. Die die Seltenen Erdenelementen umfassen, bekanntlich auch insbesondere die Gruppen der Lanthanoide. Bei den Nebengruppenelementen sind insbesondere die Übergangsmetalle hervorzuheben, sowie 1-8 Nebengruppe, I-VIII Hauptgruppe. Nach neueren Nomenkultur des Periodensystems der Elemente. Der verwendbare Ausdruck "Lösung" ist dem Fachmann ohne weiteres bekannt und sollte hier möglichst umfassend verstanden werden. Selbstverständlich werden die Metallionen bzw. Metallkomplexe erfindungsgemäß in einer Form bereit gestellt, in der sie möglichst leicht in das poröse Keramikmaterial eindringen können. Deshalb wird es sich hier in der Regel eine (flüssige) Lösung bzw. um ein homogenes Gemisch eines entsprechenden Feststoffen in der Lösung um die Porositäten des Keramikkörpers zu beladen. Nach Trocknung der Nitrate in den Porositäten entwickelt sich ein Kristall, das durch eine gesonderte Wärmebehandlung auch Oxidationsstufen durchlaufen kann. So kann auf das stabilisierte Zirkonpulver des Rohlings eine zweite Dotierung mit weiteren Stabilisatoren auf kalziniert werden. Bei dem erfindungsgemäßen Verfahren, die besonders bevorzugt einsetzbar sind, Suspension oder insbesondere Lösungen, die Metallionen der Metallkomplexe mit mindestens einem Element der aufgeführten Elemente
1. Fe(No₃)₃ ·9 H₂O
2. Cr((No₃)₃ ·9H₂O
3. Er(No₃)_{3 ·} 5 H₂O
4. Ce(No₃)₃ · 6 H₂O
5. Al (No₃)₃ · 9 H₂O
6. Ni(No₃)₂ · 6 H₂O
7. Mn(No₃) · 4 H₂O
8. Pr(No₃)₃ ·6 H₂O
9. Y(No₃)₃ · 6 H₂O
10.Co(No₃)₂· 6 H₂O
11.ZrO(No₃)₂ ·x H₂O
12.Sm(No₃)₃ ·6 H₂O
13.Nd(No₃)₃ ·6 H₂O
14. Eu(No₃)₃ · 5 H₂O
15. Dy(No₃)₃ ·x H₂O
16.Yb(No₃)₃· 5 H₂O
17.Ti(No₃)₄ ·4 H₂O
18. Bi(No₃)₃ ·5 H₂O
19.Au Cl
20.Sr(No₃)₂
21.Mg(NO₃)₂ ·6 H₂O
22.La(No₃)_{3 ·}6 H₂O
23.Ag No₃·
24.In(No₃)₃ ·X H₂O
25.Cd(No₃)₂ ·4 H₂O
26.V(No₃)₂
27.Zn(No₃)₂ · 6 H₂O
28.Dy(No₃)₃ · xH₂O
29.Tb(No₃)₃ · 5 H2O
30.Ca(No₃)_{2 ·} 4H₂O
31.C₄H₄ NNbo₉ · x H₂O
32.Pb(No₃)₂
33.Nb(No₃)₃ · 5H₂O
34.Hf(No₃)₄
35.Zr (So₄)₂ · H₂O
36.Gd(No₂)₃ · 6 H₂O
37.Sc(No₃)₃
38.Ga(No₃)₃ · xH₂O
39.Cu(No₃)_{2 ·} xH₂O
40.V₂ O₅
41.In(No₃)_{3 ·} xH₂O
42. Zr(No₃)₄
43. Na₂ SiO₃
44. Na2 O₃ Si · 9H₂O
enthalten.

Der Beladungskörperstoff dient dann als Farblösungsmittelspeicher und Speicherstabillisatorenlösung zur vollständigen Beladung aller Porositäten der porösen Keramik. Unter farbgebenden Komponenten der Farbpigmentlösung und/oder Stabilisatorenlösungen versteht man alle farbgebenden und nicht farbgebenden Komponenten die zu gewünschten und spannungsfreien sinterbaren Keramikrohling führt. Unter chemischen Stabilisatoren versteht man alle Stabilisatoren die das Zirkonoxid in ihren technischen Parametern beeinflussen können.

Erfindungsgemäß findet der gewünschte Verteilungssteuerungsschritt der farbgebenden Konzentrationen organischer oder anorganischer Salze in einem steuerbaren Abdichtung- und/oder Isolierungsmittel z.B. einer Form statt. Vorzugsweise kann dies aus einer z.B. Silikonform hergestellt werden. Erstaunlicherweise wurde entdeckt, dass auch in einer porösen Keramik die sich in einem z.B. Silikongehäuse befindet ein Verteilungssteuerungsschritt stattfindet, der der Konvektion eines Fluiden in einem Gefäß ähnlich ist. Bei dem erfindungsgemäßen Verfahren können alle Konvektionen Anwendung finden, vor einer chemischen Konvektion. Bei einer Lösung auch solutale Konvektion, bei der in Verwendung stehenden Salzlösung, auch haline Konvektion, sowie auch von der thermohaline Konvektion, sowie Mahagoni-Konvektion und elektrischen Konvention. Die Konvektion ist durch die Stoffeigenschaften, Formkörper, beeinflussten Strömungen, Energie, Entropie, Stoffe und elektrische Ladungen werden ausgetauscht unter anderem durch Diffusion, Fasenübergänge, Trocknung, Sorption, Verdampfen, Erstarrungen, Dissoziation, lissoziation Reibungen. Desweiteren kann eine Fläche als Katalysator wirken. Aus diesen Gründen ist die Konvektion auch schwer zu berechnen. Man hat durch viele hunderte Versuche und auch tausende Möglichkeiten ein präzise wirkenden Verteilungssteuerungsschritt durch die haline Konvektion und deren Einstellungen herausgefunden. Die Konvektion aus Gravitation und Dichtunterschieden wird weiterhin gesteuert durch die Menge organischer nichtorganischer Salze sowie Temperaturunterschiede elektrostatische Felder und um den umgebenden Luftfeuchtigkeitsgehalt. Sowie der Ausbildung der offenen oder abgedeckten Flächendes des porösen Keramikrohling. Der poröse Keramikrohling kann rund, kann Höhen 10-50 mm oder einen Durchmesser von 10-150 mm haben oder Hufeisenformen besitzen oder der vergrößerten Form eines gesamten Kiefers entsprechend. Sowie materialsparende Formen und Aussparungen besitzen.

Zum Transport des Fluid der steuerbaren Farbpigmentlösung und/oder reiner Stabilisatorenlösungen wird erfindungsgemäß Wasser oder eine Mischung aus Wasser mit einem organischen, insbesondere einem polaren organischen Lösungsmittel verwendet. Die farbgebenden Komponenten und/oder brandfesten Pigmente und/oder Oxide und/oder färbenden und fluoreszierenden Metalloxide und/oder organischen oder anorganischen Salze der Zirkonoxid stabilisierenden Gruppe enthalten, und/oder erfolgt durch eine wässrige Lösung oder Destillat auf Alkoholbasis. Organische Lösungsmittel sind zum Beispiel aliphatische Alkohole. Das Lösungsmittel und/oder Transportfluid kann gegebenenfalls Additive, wie zum Beispiel Stabilisatoren oder Elektrolyte, Komplexbildner, Dispergiermittel usw., beinhalten. Die Additive sind entweder im Beladungskörperstoff oder in Farbpigmentlösung und/oder Farbpigmentstabilisatorenlösung enthalten. Unter einer Farbpigmentlösung und/oder Farbpigmentstabilisatorlösung versteht man auch alle Lösungen die in einer porösen Keramik in den Porositäten befindlich sind und sich durch offene und geschlossene Flächen am porösen Keramikrohling gesteuert verteilen.

Es ist außerdem zweckmäßig, dass in die Farbpigmentlösung und/oder Farbpigment Stabilisatorlösung chemische Stabilisatoren eingebracht werden, wie z.B. Cerchlorid, Cernitrat, oder Ammonium Chernitrat eingebracht wird. Dieses z.B. Cerchlorid wird bei einer Temperatur von ca. 60°-110° in einer Lösung mit einem pH Wert von 5-9 durch ein Oxidationsmittel zu Ceroxid, das in den Poren des vorgefertigten Zirkonrohlings kalziniert, wird und sich so homogen mit dem Zirkonoxidgitter verbindet. Das gleich gilt für Yttrium und/oder Zirkonnitrate die das Zirkonoxid stabilisieren können und deren Verbindungen. So entsteht in den Beladungs- und Verteilungssteuerungsschritten nicht nur eine Farbgebung, sondern auch die erfinderische Möglichkeit für physikalische Eigenschaften der Zirkonoxidkeramik mit entsprechenden Stabilisatoren in gewünschte Zonen der vorgefertigten Zirkonrohlings einzubringen. Zusatzstoffe und Oxidationsmittel sind Aluminiumnitrat, Natriumhydroxid, Kaliumhydrooxid, Wasserstoffperoxid Soidsalzen, Zirconium (IV) Oxynitrate, Hydriumnitrate, Hypochloridsäure Natriumhypochlorit, Calciumhypochlorit und der gleichen. Erfindungsgemäß lassen sich so mittlere Partikelgrößen von ca. 0,01µ bis 0,5µ und ein Kristalldurchmesser von ca. 1bis 80 nm z.B. von Ceroxid in den Poren der vorgefertigten porösen Keramik herstellen, in einem ersten oder mehreren Beladungsschritten. Ebenfalls ist es zweckmäßig, dass in die Farbpigmentlösung und/oder Farbpigmentstabilisatorlösung Öle und/oder Benzine eingebracht werden. Sowie das eine Strömungsreduzierung und Filter zwischen Beladungskörperstoffspeicher und z.B. Silikongehäuse zu legen. Der kapillare Körper ist dann Strömungsreduzierungsfilter und sorgt erstens dafür, das möglichst wenig Konzentrationsansammlung im den porösen Keramikrohling gelangen und sorgt zweitens somit für eine Kapillarunterdruckbeladung. Da die Auflagenflächen der porösen Keramik immer mit Zirkonstaub behaftet sind, was die Farbpigmentlösung und/oder Farbpigmentstabilisatorenlösung verunreinigt, so das Beladungskörperstoffe und/oder kapillare Strömungsreduzierung und Filter und Beladungskörperstoffspeicher sind.

Bei einer möglichen erfindungsgemäßen Verfahrensvariante werden die Farbpigmentlösung und/oder Farbpigmentstabilisatorenlösung und/oder brandfesten Pigmente und/oder Oxide und/oder färbende und fluoreszierende Metalloxide und/oder organischen oder anorganischen Salze in/unter einer Vakuumatmosphäre und /oder inerte Gas (Argon) ins Konvektionsgehäuse beladen. Das Transportieren der farbgebenden Komponenten und/oder brandfesten Pigmente und/oder Oxide und/oder färbenden und fluoreszierenden Metalloxide und/oder organischen oder anorganischen Salze der Farbpigmentlösung und/oder Stabilisatorenlösungen kann auch unter einer annähernden Vakuumatmosphäre stattfinden. Dies ist jedoch nicht zwingend erforderlich. Zu starkes Vakuum kann je nach Porosität des keramischen Rohlings zu nicht gefühlten Porositäten und/oder zu ungewünschten Konzentrationsansammlungen der farbgebenden Komponenten führen.

Bei einer weiteren erfindungsgemäßen Verfahrensvariante werden die Farbpigmentlösung und/oder Stabilisatorenlösungen und/oder brandfesten Pigmente und/oder Oxide und/oder färbenden und fluoreszierenden Metalloxide und/oder organischen oder anorganischen Salze der haline Konvektionen unter Druck transportiert. Somit kann auch der Kapillardruck durch Druckbeaufschlagung der kapillardruckenthaltenden Beladungskörperstofflösungsmittelvorrichtung erhöht werden. Zu viel Druck kann zur Über-und Unterschlagung von Porositäten und deren Hohlräumen führen und starke Verstopfungen verursachen und somit zu ungewünschten Konzentrationsansammlungen der farbgebenden Komponenten führen. Unter der kapillardruckenthaltenden Beladungskörperstoffllösungsmittelvorrichtung versteht man alle dichten Auflagen oder Aufnahmevorrichtungen in den oder auf denen, sich Lösungen mit Farb- und nicht farbgebenden und/oder chemischen Stabilisatoren Komponenten unter Erhöhung und/oder Reduzierung des Kapillardrucks in den Porositäten der Keramik speichern lassen.

Bei dem erfindungsgemäßen Verfahren werden die_Farbpigmentlösung und/oder Farbpigmentstabilisatorenlösung mit und/oder brandfesten Pigmente und/oder Oxide und/oder färbenden und fluoreszierenden Metalloxide und/oder organischen oder anorganischen Salze und/oder farbgebenden chemischen Stabilisatoren in die Poren der porösen Keramik mit einem Beladungskörperstoffe unter kapillardruckhaltenden Farblösungsmitteln beladen und/oder Kapillarunterdruckbeladung eingebracht. An der Oberfläche der Keramik sind Porenöffnungen vorhanden, so dass die farbgebenden Komponenten und/oder brandfesten Pigmente und/oder Oxide und/oder färbenden und fluoreszierenden Metalloxide und/oder organischen oder anorganischen Salze und/oder farbgebenden chemischen Stabilisatoren in diese eindringen. Die Eindringtiefe ist abhängig von der Menge der Farbpigmentlösung und/oder Farbpigmentstabilisatorenlösung und eingestelltem Kapillardruck, der kapillardruckenthaltende Beladungskörperstofflösungsmittelvorrichtung des Kapillardrucks. Der kapillardruckenthaltende Beladungskörperstofflösungsmittelvorrichtung sollte niedriger sein. Als der Kapillardruck der porösen Keramik und gleichmäßig und nicht zu schnell und ohne Lufteinschlüsse alle Porositäten mit Farbpigmentlösung und/oder Farbpigmentstabilisatorenlösung beladen werden.

Zweckmäßigerweise werden die Beladungskörperstoffe mit farbgebenden Komponenten gefüllt und als Beladungskörperstoffe lösungsmittelspeicher verwendet, die als einfaches Lager der Farbmischungen oder als Filter dienen. Wie z.B. Bierdeckel enthalten dann die gewünschten Konzentrationen und farbgebenden Komponenten und/oder chemischen Stabilisatoren der Zahnfarbe. Weiterhin beinhalten kapillardruckhaltende Beladungskörper die Komplexlösungen (Komplexlösungen sind alle Salze der seltenen Erdegruppe usw.) .Durch Auflegen der Bierdeckel auf die poröse Fläche des porösen keramischen Rohlings der sich in dem z.B. Silikongehäuse befindet. Das z.B. Silikongehäuse wird nun mit poröser Keramik auf die kapillardruckenthaltende Beladungskörperstofflösungsmittelvorrichtung flächig aufgelegt. So kann nun die Farbpigmentlösung und/oder Farbpigmentstabilisatorenlösung an beliebige Stellen im porösen Keramikblock beladen werden. Unter Beladung versteht man, alle Benetzungsarten und Mengen, die mindestens eine Ionenbelegung auf keramischen Partikeln in gepresster Rohlingsform unter Nutzung der Hohlräume zwischen den verschiedenen Keramikpartikeln bewirken. Unter Beladungskörperstoffen versteht man alle Materialien und Stoffe die flüssigkeitsdurchlässig sind und/oder strömungsreduzieren und/oder Kapillardruck halten können, in Bezug auf die poröse Keramik.

Das erfindungsgemäße Verfahren bietet außerdem die Möglichkeit, dass die gleichmäßige und/oder Konzentrationsverteilung der Verteilungsströmungsschritt wird durch das z.B. Silikongehäuse Nitratgehalt, Temperatur, Luftfeuchtigkeitsgehalt und mit verschiedenen großen Flächen die mit umgebender Luftfeuchtigkeit in Berührung stehen, gesteuert. Es ist dabei zu achten, dass die umgebende Luftfeuchtigkeit nicht unter ca. 30% sinkt, bei Temperaturen um ca. 25°. Da ansonsten die Bewegungsrichtung der Farbpigmente und/oder Zirkonoxidstabilisatoren zu einer sehr starken Konzentrationsansammlung führen, die zu einer starken sehr Konzentrationsansammlung führt. Diese Beobachtung ist aber abhängig von der jeweiligen vorhandenen Porosität des Keramikkörpers.

Während des erfindungsgemäßen Verfahrens ist es vorteilhaft, wenn Farbmessungen an natürlichen Zähnen in einem programmierbaren Speicher hinterlegt werden, so dass die Farbe der natürlichen Zähne mit dem entsprechenden Farbpigmentlösung und/oder Farbpigmentstabilisatorenlösung reproduziert werden kann. Die Zahnfarbe der natürlichen Zähne des Patienten wird demnach gemessen. Anschließend werden die ermittelten Farbdaten in einer Software oder in einem programmierbaren Speicher gespeichert. Die Software oder der programmierbare Speicher kann mittels der Farbdaten die natürliche Zahnfarbe mit Farbpigmentlösung und/oder Farbpigmentstabilisatorenlösung berechnen bzw. reproduzieren. Der Farbverlauf wird dann graphisch vom CAD/CAM System angezeigt wird. So kann der Kunde im Dentallabor seinen Farb- und physikalischen Parameter digital zusammenstellen und diesbezügliche eine Bestellung auslösen.

Des Weiteren wurde erstaunlicher Weise entdeckt, dass durch Abdeckungen bzw. Abkleben von längslaufende Zebrastreifen unter nicht abgeklebten Zebrastreifen der porösen Keramik im Keramikblock eine verlaufende räumliche Farbkonzentrationswelle und/oder Stabilisatorenkonzentrationswellen bei offenen oder kreisrunden Abklebungen ein räumliche Farbkonzentrationskonuskegel und/oder Stabilisatorenkonzentrationswellen entsteht. Wenn man die gewünschten Felder mit verschiedenen hohen Rahmen umgibt, stellt sich im Rahmen selbst eine höhere Luftfeuchtigkeit ein, obwohl eine konstante Luftfeuchtigkeit vorhanden ist. Die Flächen mit hohem Rahmen und höherer Luftfeuchtigkeit bilden eine vertikal verlaufende höhere räumliche Farbkonzentration mit und/oder chemischen Stabilisatoren. Flächen mit niedrigem Rahmen und niedriger Luftfeuchtigkeit bilden eine farbintensivere und niedrigere räumliche Farbkonzentration mit und/oder chemischen Stabilisatoren des Zirkonoxid.

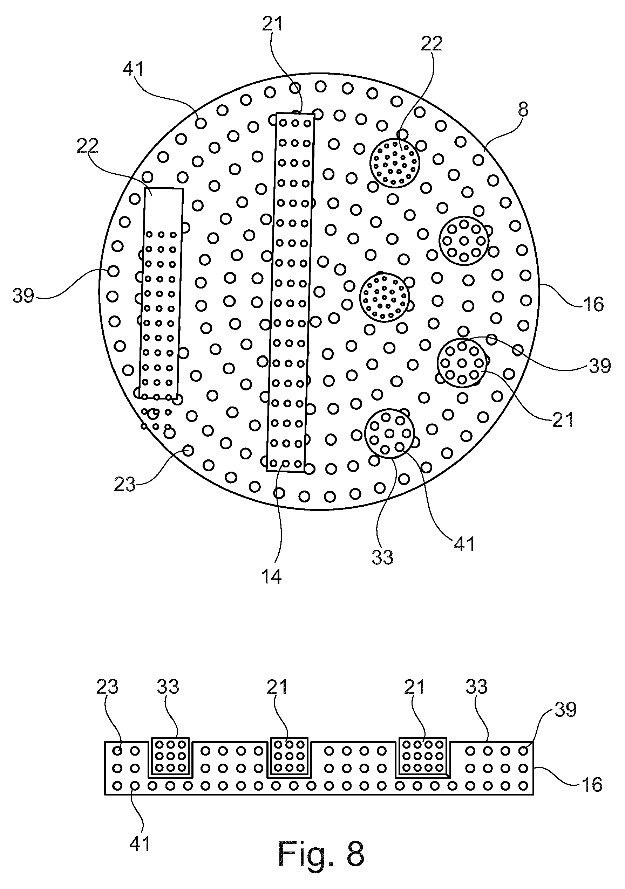

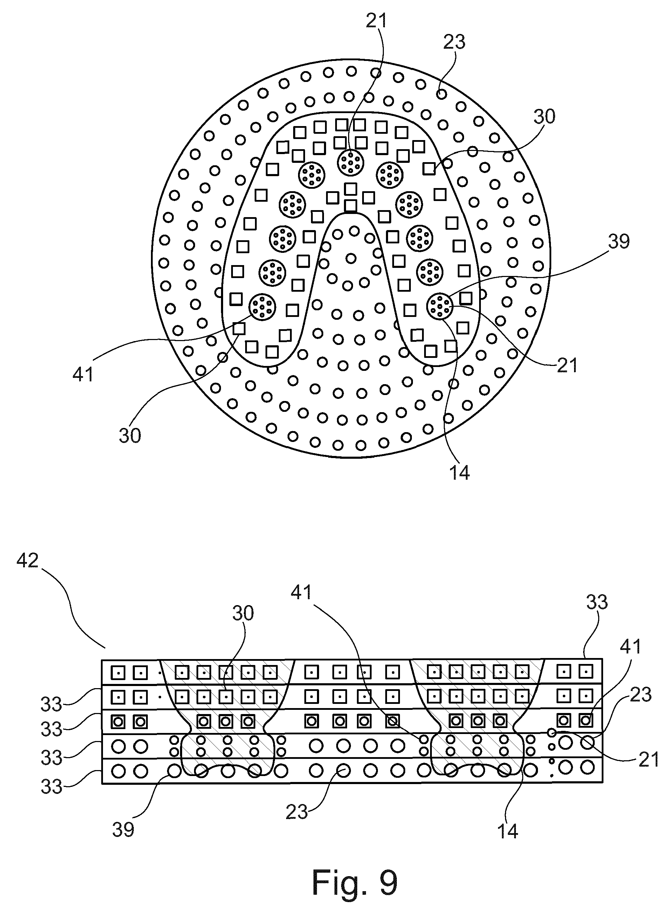

Eine weitere Möglichkeit des Verfahrens der Erfindung ist, dass eine programmgesteuerte Maschine oder Gerät, insbesondere eine CAD/CAM-Anlage, aus den zur Verfügung stehenden Konzentrationszonen Felder der Keramikrohlinge die Farbkonzentration oder Stabilisatoren heraussucht und herausfräst, die der Zahnfarbe der natürlichen Zähne entsprechen und die gewünschten physikalische Parameter haben z.B. bezüglich einer geringeren Härte im wichtigen Okklusionsbereich aufweisen. Weiterhin kann der Beladungskörperstoffe ausgefräst werden, zu einer gewünschten farbverlaufenden Figurgebung siehe Fig. 8 und Fig.9 oder die Anatomie der Oberfläche eines Zahnersatzes haben. Erstaunlicherweise stellte sich heraus, dass durch das erfinderische Verfahren, welches die Porosität des keramischen Rohlings verringert, gleichzeitig die Kantenstabilität gesteigert wird. Dies ist besonders vorteilhaft in Verbindung mit 3D-gedruckten Keramik-Strukturen, die eine sehr hohe Porosität aufweisen und daher nur schwer fräsbar sind. So kann die Qualität der Fräsung z.B. an dünnen Kronenrändern zum Kantenausbrechen führen. Die Fräszeiten der CAD-CAM System können somit um ca. 25% gesteigert werden, was zu einem höheren Maß der Wirtschaftlichkeit und Effizienz der teuren CAD-CAM Systeme und ihrer Amortisation beiträgt. Dadurch gekennzeichnet, dass durch Beladung und einem Verteilungssteuerungsschritt die Kantenstabilität und Festigkeit verbessert wird und somit auch die Fräßgeschwindigkeit um ca. 25% gesteigert werden kann.

Es werden des Weiteren erfindungsgemäß die farbgebenden Farben gespeichert der durchgeführten Farbmessung an die programmgesteuerte Maschine oder an das programmgesteuerte Gerät übermittelt. Die zuvor ermittelten Farbdaten der natürlichen Zähne des Patienten werden an die programmgesteuerte Maschine oder Gerät übermittelt, so dass die Maschine einen oder mehrere Beladungskörperstoffe verwendet, und diese in ein z.B. Silikongehäuse einlegt, um und/oder die Zahnfarbe und/oder die Härte der natürlichen Zähne des Patienten herzustellen.

Bei einer vorteilhaften Verfahrensvariante werden ganze vorgesinterte und/oder vorgepresste Keramikblöcke vor der CAD/CAM Bearbeitung eingefärbt. Das bedeutet, dass Keramikblöcke bereits in unterschiedlichen Grundfarben und/oder Materialzusammensetzungen und der gewünschten Dichte hergestellt wird, damit nur noch die Stabilisatoren z.B. Wellen und/oder räumliche Stabilisatorenkonzentrationskegel in gewünschte Flächen mit einem Verteilungsgesteuertem Schritt entstehen kann oder eine erhöhte Rissfähigkeit oder eine verringerte Härte eingestellt wird. Vorteilehafte poröse Keramiken sind zurzeit 3Y-TZP, kubische 5Y-TZPund Cer-TZP.

Verfahren zur Herstellung eines polychromen und/oder räumlich polychromen und/oder eines monochrom gefärbten vorgefertigten keramische Körper insbesondere eines derartigen Dentalkeramikrohlings dadurch gekennzeichnet, dass die vorhandenen Restporositäten eines vorgefertigten keramischen Rohlings durch Beladung und/oder Verteilungssteuerungsschritt mit Pigmentlösungen und Stabilisatoren nach der Trocknung eine Gewichtszunahme von ca. 0,01 - 25% Gewichtsprozent aufzeigt. Für eine Verfahrensausführungsform wird eine porös gebrannte keramischer Weißling oder eine porös gepresste Glaskeramik als Keramik verwendet. Das Verfahren ist bei allen porösen Keramiken anwendbar. Unabhängig ob es sich dabei um eine gepresste, gebrannte, gebinderte, entbinderte und/oder angesinterte Keramik handelt. Die Keramik muss jedoch Poren aufweisen, so dass die farbgebenden Komponenten und/oder brandfesten Pigmente und/oder Oxide und/oder färbenden und fluoreszierenden Metalloxide und/oder organischen oder anorganischen Salze in mindestens einer Bewegungsrichtung der Farbpigmente und/oder Stabilisatoren zulassen.

Verfahren zur Herstellung eines polychromen und/oder räumlich polychromen und/oder eines monochrom gefärbten vorgefertigten keramischen Körper insbesondere derart eines Dentalkeramikrohlings, dass mit den Beladungs-und/oder Verteilungsschritt die Härte und/oder Rissfähigkeit in Abhängigkeit des chemischen Stabilisators eingestellt wird, die sich in den Porositäten des vorgefertigten keramischen Rohlings befinden. Bei dem Verfahren wird beispielsweise eine poröse Keramik aus Lanthanoxid (La₂O₃), Siliciumdioxid (SiO₂), Leucit, Vanadium(V)-oxid (V₂O₅), Ceroxid, Erbiumoxid Zirkonoxid (ZrO₂), Yttriumoxid (Y₂O₃), Hafniumoxid (HfO₇), Aluminiumoxid (Al₂O₃) Phosphoroxid (P₂O₃, P₂O₄, P₄O₁₀), Titanoxid (TiO₂), Zinnoxid (SnO, Sn₂O₃, SnO₂), Boroxid ((BO)ₓ, (B₂O)ₓ), Bortrioxid (B₂O₃), Fluor (F₂), Natriumoxid (Na₂O), Bariumoxid (BaO), Strontiumoxid (SrO), Strontiumperoxid (SrO₂), Magnesiumoxid (MgO), Zinkoxid (ZnO), Zinnoxid (SnO, Sn₂O₃, SnO₂), Calciumoxid (CaO), Titanoxid (TiO₂), Nioboxid (NbO, NbO₂, Nb₂O₅), Tantaloxid (TaO, TaO₂, Ta₂O₅), dotierte Spinelle und/oder weitere Oxide sowie Mischungen hiervon umfasst, verwendet.

Verfahren zur Herstellung eines polychromen und/oder räumlich polychromen und/oder eines monochrom gefärbten vorgefertigten keramischen Körpers insbesondere derart eines Dentalkeramikrohlings, dadurch gekennzeichnet, dass in den Porositäten nach Trocknen der Farbpigmentlösung und Stabilisatoren die sich in den Porositäten des Keramikkörpers befinden. Eine Oxidationsphase der Dotierung bei ca.100° bis 800° und/oder einer anschließenden Kalzinierungsphase bei ca. 800°-1500° für ca. 1-24 Stunden stattfindet. Des Weiteren kann bei dem erfindungsgemäßen Verfahren Feldspaltkeramik, Zirkonoxid- und/oder Leucitverstärkte-Keramik, Lithiumsilikat-Gläser oder Lithiumsilikat-Glaskeramik oder Lithiumdisilikat-Glaskeramik, Silikatkeramik und/oder Oxidkeramik eingefärbt werden. Als einzufärbende Keramik für das Verfahren kann jede Oxidkeramik oder eine Keramik auf Basis von Oxidkeramik verwendet werden. Oxidkeramiken sind Hochleistungskeramiken, die härter, verschleißfester, wärmebeständiger und spröder als Hartmetalle sind. Sie bieten demnach die wichtigsten Eigenschaften für eine Implantatprothetik oder ein Implantat und/oder einen Zahnersatz. Des Weiteren kann auch eine Keramik, die auf Basis von Glaskeramik und/oder Glas besteht, verwendet werden.

Verfahren zur Herstellung eines polychromen und/oder räumlich polychromen und/oder eines monochrom gefärbten vorgefertigten keramischen Körpers insbesondere derart eines Dentalkeramikrohlings, dass dadurch gekennzeichnet das ein handelsüblicher 3Y-TZP verwendet wird mit Beladungs- und Verteilungsschritt Farbpigmente und Stabilisatoren eingebracht werden um aus einem 3Y-TZP ein CE-TZP herzustellen. Bei einem erfindungsgemäßen Verfahrensschritt wird die farbgebende Komponente in den porösen Keramikrohlingen unter ansteigender Vakuumatmosphäre oder in einem sauerstofffreien oder annähernd sauerstofffreien Raum gesintert, um die farbgebenden Komponenten und/oder brandfesten Pigmente und/oder Oxide und/oder färbenden und fluoreszierenden Metalloxide und/oder organischen oder anorganischen Salze in der Keramik zu oxidieren, zu kalzinieren und zu fixieren. Das bedeutet, dass die farbgebenden Komponenten und/oder brandfesten Pigemente und/oder Oxide und/oder färbenden und fluoreszierenden Metalloxide und/oder organischen oder anorganischen Salze in den Poren der Keramik durch einen Sintervorgang oxidiert 'kalziniert und fixiert werden, und/oder in ein Oxid verwandelt werden oder direkt in einem Endsintervorgang. Unter einem räumlich polychromen keramischen Körper versteht man auch die Transparenz die durch z.B. die einzelne Kristalle aus polykristallines Körper erzielt werden, z.B. das die kubischen Kristalle des Zirkoniumoxid durch z.B. Yttriumoxid (Y-KZP oder mit Calciumoxid) (CSZ) stabilisiert sind. Da die kubischen Kristalle keine optischen Anisotropie haben und somit eine besonders hohe Transparenz generieren. Diese können durch das erfindungsgemäße Verfahren des Verteilungsteuerungsschritt homogen und/oder konzertiert in den Poren des keramischen Rohlings eingefärbt oder in einen oder mehreren Farbtonkonzentrationen verlaufend eingefärbt werden, auch je nachdem wie der keramische Rohling zusammengesetzt ist. So sind Inlinle - Transmissionen Wellenlängen(600nm) Probenstärke x30mm x 1 mm von 35% T bei Wellenlänge (600nm) bis 95% T bei Wellenlänge (600mn) einstellbar. Sowie der Lichtbrechungsindex von 1,20 bis 2,20, besonders bevorzugt 1,60 bis 1,65 für die dentale Anwendung. Die Transluzenzeinstellungen erfolgen mit mindestens einem Element z.B.
Er(No₃)₃ · 5 H₂O
Ce(No₃)₃ · 6 H₂O
Al (No₃)₃ · 9 H₂O
Y(No₃)₃ · 6 H₂O
Nd(No₃)₃ · 6 H₂O
Sr(No₃)₂
Mg(No₃)₂ · 6 H₂O
La(No₃)₃ · 6 H₂O
Zn(No₃)₂ · 6 H₂O
Ca(No₃)₂ · xH₂O
Ga(No₃)₃ · xH₂O
Cu(No₃)₂ · xH₂O
Zr(No₃)₄
Na₂ SiO₃
Na2 O₃ Si · 9H2O
in Gew.-% bezogen auf die Keramik von 0,001 bis 15 Gew.-% je nachdem wie der keramische Rohling zusammengesetzt ist. Die Chromatizität L *a* b* Kolorimetrie System Angaben kann eine Farbe jedes Punktes zu einem anderen Punkt enthalten werden. Je nachdem welche Zusammensetzung und Einfärbung der keramische Rohling hat. Im Fall eines Dentalmaterials gerichtete Anspruch ist L* im Bereich 40 bis 90 enthalten, a* 35 bis -35 enthalten, b* -15 bis 40 enthalten. Bei einer z.B. endgesinterten Keramik von 1,5mm Stärke und Reverenzhintergrund von L*94 a*3,87 b*-12,85 weisem Hintergrund.

Verfahren zur Herstellung eines polychromen und/oder räumlich polychromen und/oder eines monochrom gefärbten vorgefertigten keramischen Rohlings mit vorteilhaften physikalischen Parametern dadurch gekennzeichnet, dass 0,001 bis 50 Gew.-% bevorzugt 1,5 und 50 Gew.-% bezogen auf das keramische Gewicht. Stabilisatoren jeweilig relativ zum Zirkonoxidgehalt in den Porositäten des keramischen Körpers beladen und/oder im Verteilungssteuerungsschritt konzentriert an gewünschten Zonen homogen und/oder konzentriert verteilt wird. Zur Anwendung vorteilhafter poröser Keramiken sind dann z.B. Y-KZP, CSZ, TZP, 3Y-TZP, 5Y-TZP, TZP, SSZ, PSZ, ATZ , CETZP, KSZ, ZrSio4, ZrSi2, und Mischungen aus den Pulvern. Z.B. kann ein Zirkoniumnitrat zur Herstellung von reinem Zirkon IV Oxidschichten verwendet werden. oder Zirkonium mit Silicium Tetrachlorid bilden sich bei der Reaktion von Zirkoniumoxid mit Silizium. 3Si + Zro₂ → Zr Si_{2 +}Si o₂- Zr Si₂. Ebenso kann eine Verbindung durch Fusion mit Si o₂- Zr o₂ durch die Verwendung eines Zirkoniumsalzes mit Umsetzen von Natriumsilikat in einer wässrigen Lösung erfolgt zu (Zr Sio₄₎. Somit implementiert das erfindungsgemäße Herstellungsverfahren auch alle Möglichkeiten vorteilhafter Zirkoniumverbindungen anzuwenden. Das erfindungsgemäße Verfahren umfasst folgende Schritte:a Bereitstellen einer durchgängig porösen und/oder vorgesinterten und/oder entbinderten und/oder gebinderten Keramik, insbesondere eines Keramikkörpers für die CAD/CAM Bearbeitung
(1) a Bereitstellen der Keramik
(2) b Abdichtung und/oder Isolierung mittels einer Form des Keramikkörpers
(3) c Beladung der Porositäten des Keramikkörpers, wobei die Schritte (2) b und (3) c in beliebiger Reihenfolge ausgeführt werden können
(4) d Verteilungssteuerungsschritt der farbgebenden und/oder oxid- gebenden stabilisatorischen Elemente
(5) e Flüssigkeitsentzug bis zum Ende der Verteilungssteuerung in den abgedichteten und/oder isolierten Keramikkörper
(6) f Trocknen des keramischen Rohlings und/oder Ent- oder Ansinterung und/oder zur Fixierung der Salze zur Oxidationphasenbildung und Oxidation zur Kalzinierung und/oder zur Endsinterung
(7) g CAD/CAM Bearbeitung
(8) h Endsintern und/oder Sintern zur Fixierung der Oxide in einem Sinterprogramm

Verfahren zur Herstellung eines polychromen und/oder räumlich polychromen und/oder eines monochrom gefärbten vorgefertigten keramischen Körpers insbesondere derart eines Dentalkeramikrohlings, mit vorteilhaften einstellbaren technischen Parametern dadurch gekennzeichnet ist, dass durch Beladung und/oder Verteilungsschritt nur stabilisierenden Lösungen in den Poren des vorgefertigten keramischen Körpers eingebracht werden. In der erfindungsgemäßen Farbpigmentlösung und/oder chemischen stabilisatorischen Lösung sind Konzentrationen der farbgebenden Komponenten und/oder brandfesten Pigmente und/oder Oxide und/oder färbenden und fluoreszierenden Metalloxide und/oder organischen oder anorganischen Salze der in späteren Oxidation und/oder Sinterprozess entstehen Zirkonstabilisatoren, Oxide zwischen 0,001 Gew.-% und 50 - 90 Gew.-% oder zwischen 0,0001 Gew.-% und 10 Gew.-% bezogen auf das Keramikgewicht enthalten. Je nachdem welche Farbton erreicht oder welche physikalischen Parameter eingestellt werden sollen, sind entweder geringe oder größere Mengen an farbgebenden Komponenten und/ oder stabilisatorischen Komponenten und/oder brandfesten Pigmenten und/oder Oxiden und/oder färbenden und fluoreszierenden Metalloxiden und/oder organischen und/oder anorganischen Salzen erforderlich. Demnach sind die Konzentrationen der farbgebenden Komponenten und chemischen Stabilisatoren und/oder brandfesten Pigmente und/oder Oxide und/oder färbenden und fluoreszierenden Metalloxide und/oder organischen oder anorganischen Salze auch in den Beladungskörperstoffen um die gewünschte Farbe und/oder physikalische Parameter zu erzeugen und zu speichern.

Erfindungsgemäß beinhalten die Farbpigmentlösung und/oder chemischen Stabilisatoren in Lösung und/oder der Beladungskörperlösungsmittelspeicher und/oder die Beladungskörperstoffe Konzentrationen aller farbgebenden und nicht farbgebenden Komponenten die in der Verteilungssteuerungsschritt auch chemische Stabilisatoren in Lösung des Zirkonoxid bewegbar sind, einsetzbar.

Die farbgebende Lösung wird beim Verteilungssteuerungsschritt stabil gelöst, um zu verhindern, dass aus gemischten Elementen der Farbpigmentlösung bei dem Verteilungssteuerungsschritt einzelne Elemente aus der Farbpigmentlösung und/oder chemische Stabilisatorenlösungen im Verteilungssteuerungsschritt in der Porosität des vorgefertigten Rohlings einzelne Elemente aus der der Farbpigmentlösung und/oder Farbpigmentstabilisatorenlösung in eine Bewegungsrichtung zer- und/oder absetzten können. Dazu eignen sich Vorauflösungen aus Z.B.
Zro(No₃)₂ · x H₂O
Ce(No₃)₃ · 6 H₂O
Al(No₃)₃ · 9 H2O
Zr(No₃)₄
Y(No₃)_{3 ·} 6 H₂O
Sm(No₃)₃ ·6 H₂O
Ca(No₃)₂ · xH₂O
Gd(No₂)₃ · 6 H₂O und/oder andere Salze.

Erfindungsgemäß beinhalten alle bekannten farbgebenden Komponenten organische und anorganische Salze, die anwendbar sind und beim Endsinterbrand sich in ein Oxid verwandeln und/oder die chemischen Stabilisatoren in Lösung für physikalischen Parameter einer Zirkonoxidkeramik. Diese Bestandteile können zu einem als Farbadditiv, Stabilisator und zu anderen als Sinterhilfsmittel fungieren. Die löslichen Bestandteile können in das Kristallgitter eingebaut d.d. substituiert werden oder in Form von Verbindungen zum B. Mischkristallen, in der Korngrenzenphase abgeschieden sein. Sowie ganz neue Teile von stabilisiertem Zirkonkristallen oder Mischkristallen in den Porositäten des vorgefertigten Keramikkörperrohlings bilden. Da hier auch die Umgebung auf die Kristallbildung Einfluss hat. Da dies noch nicht erforscht ist, werden weitere noch nicht bekannte physikalische Eigenschaften in der Zukunft gefunden werden. Da auch die Wissenschaft bis heute keine Erklärung für das Verhalten der Fasenumwandlung des Zirkons durch die Stabilisatoren hat. Die wichtigen physikalischen Eigenschaften der von Y3-TZP, Y5-kubisch TZP, Ce-TZP können so jeweils abhängig vom beladen des porösen keramische Rohlings und gewünschter Anwendung (Gerüst oder monolithischer Zahnersatz) die Vickershärte Hv10 von 400 - 1650, die Rissfähigkeit MPa.m von 4,5-16,5 aufweisen und räumlich und polychrom eingestellt werden. Somit lassen sich mit aufgeführten Keramiken durch mindestens ein Element z.B.
Zro(No₃)₂ · x H₂O
Ce(No₃)₃ · 6 H₂O
Al (No₃)₃ · 9 H₂O
Y(No₃)₃ · 6 H₂O
Sm(No₃)₃ ·6 H₂O
Ti(No₃)₄ ·4 H₂O
Sr(No₃)₂
La(No₃)₃ · 6 H₂O
V(No₃)
Zn(No₃)₂ · 6 H₂O
Ca(No₃)₂ · xH₂O
Hf(NO₃)₄
Zr(So₄)₂ · H₂O
Gd(No₂)₃ · 6 H₂O
Sc(No₃)
In einem oder mehreren Beladungsschritten physikalischen Eigenschaften in gewünschten Zonen des keramischen Rohlings festlegen. Je nachdem welche physikalischen Parameter eingestellt werden sollen. Die Stabilisatorenlösungen haben Konzentrationen von 0,001 bis 30 Gew. % bezogen auf das Gewicht des keramischen Rohlings. Die physikalischen Werte z.B. einer 70% Zirkonoxidkeramik siehe Tabelle mit beispielhaften Werten aufgeführt.

Ein weiteres erfindungsgemäßes Ausführungsbeispiel stellt ein Beladungskörper dar, das alle Formen besitzen kann , wobei die farbgebenden Komponenten und/oder brandfesten Pigmente und/oder Oxide und/oder färbenden und fluoreszierenden Metalloxide und/oder organischen oder anorganischen Salze mindestens eines der Elemente Yttrium, Eisen, Titan, Selen, Silber, Indium, Gold, Chrom, Kupfer, Praseodym, Kobalt, Nickel, Mangan, Erbium, Neodym, Cer, Aluminium, Zirkon, oder Seltenerdmetalle sowie Mischungen hiervon beinhalten, die in den Beladungskörperstofflösungsmittel eingebrachten farbgebenden Komponenten und/oder nicht farbgebenden Komponenten. Die Beladungskörper können auch unter Kapillardruck der Farbpigmentlösung und/oder Farbpigmentstabilisatorenlösung in einer luftdichten Verpackung gelagert werden.

Des Weiteren beträgt die Schichtstärke der Beladungskörper, die in und/oder auf der kapillardruckenthaltende Beladungskörperstofflösungsmittelvorrichtung und als Beladungskörperstofflösungsspeicher und Kapillarkraftreduzierung und Filter eingesetzt werden, eine Stärke zwischen 0,01 mm und 250 mm. Die Schichtstärke variiert zwischen dem oben genannten Bereich und ist aber auch abhängig vom Durchmesser der keramischen Körper, von der Porosität und Konzentration der Farbpigmentlösung und/oder Farbpigmentstabilisatorenlösung. Die Farbpigmentlösung und/oder Farbpigmentstabilisatorenlösung kann auch in den Beladungskörpern getrocknet werden, um dann auf der Beladungskörperstofflösungsspeicher und/oder kapillardruckenthaltende Beladungskörperstofflösungsmittelvorrichtung den Kapillardruck wieder aufzubauen, das zum Beladung der Farbpigmentlösung und/oder Farbpigmentstabilisatorenlösung und der Lösung der chemischen Stabilisatoren in die Porositäten führt.

Es ist ebenfalls zweckmäßig, dass die Farbpigmentlösung und/oder Farbpigmentstabilisatorenlösung mit den darin beinhalteten Füllstoffen und farbgebenden Komponenten und/oder brandfesten Pigmenten und/oder Oxiden und/oder färbenden und fluoreszierenden Metalloxiden und/oder organischen oder anorganischen Salzen der chemischen Stabilisatoren für Zirkonoxid als Beladungskörperstoff gespeichert wird. Das die Beladungskörperstoffe verschiedene geometrische Formen aufweisen, die adaptierbar sind. Das bedeutet auch, dass Farbpigmentlösung und/oder Farbpigmentstabilisatorenlösung Beladungskörperstoffe den gleichen Kapillardruck aufweisen. Die Beladungskörperstoffe können jetzt nebeneinander, übereinander oder hintereinander auf den keramischen Rohlingen adaptiert werden, ohne dass sich die Farbpigmentlösung und/oder Farbpigmentstabilisatorenlösung vermischen und das durch die Größe bzw. Volumen und Materialauswahl der Beladungskörperstoffe auch eine wunschgemäße Menge an farbgebenden Komponenten und Stabilisatoren speicherbar sind.

Es werden nicht alle Materialien und/oder Komponenten und nicht farbgebenden Komponenten beziehungsweise Stoffe aufgeführt, die in dem erfindungsgemäßen Verfahren beziehungsweise in dem erfindungsgemäßen Verteilungssteuerungsschritten verwendet werden können. Dem Fachmann sollten jedoch durch die obigen Ausführungen alle Möglichkeiten bekan Weitere Einzelheiten, Merkmale, Merkmalskombinationen und Wirkungen auf der Basis der Erfindung ergeben sich aus der nachfolgenden Beschreibung bevorzugter, beispielhafter Ausführungsformen der Erfindung sowie aus den Zeichnungen. Diese zeigen in:
- Fig. 1: Ablaufschema des erfindungsgemäßen Verfahrens,
- Fig. 2: Querschnitt monochromen Keramikrohling im z.B Silikongehäuse,
- Fig. 3: Querschnitt polychrom Keramikrohling im z.B. Silikongehäuse,
- Fig. 4: Querschnitt polychrom Keramikrohling und räumlicher Stabilisationskonuskegel oder räumlicher Stabilisationskonuskege mit und/oder chemische Stabilisatoren im Silikongehäuse,
- Fig. 5: Querschnitt polychrom und räumlicher Farbkonzentrationswelle und räumlicher Farbkonzentrationskonuskegel mit und/oder chemische Stabilisatoren des Zirkonoxid mit programmierbarer CAD/CAM Bearbeitung,
- Fig. 6: z.B. Silikongehäuse Konzentrationsausgleich in mit einem Beladungskörper,
- Fig.7: z.B. Silikongehäuse zum Konzentrationsausgleich der Farbkonzentrationen mit und/oder chemische Stabilisatoren des Zirkonoxid mit mindestens einem Beladungskörperlösungsmittelspeicher Querschnitt mit Beladungskörper,
- Fig. 8: Querschnitt Beladungskörper,
- Fig. 9: Querschnitt Beladungskörper gestapelt,
- Fig. 10: Prothesenkörper Querschnitt im Block,
- Fig. 11: Querschnitt Komplettsystem z.B. Silikongehäuse kapillardruckenthaltende Beladungskörperstoftlösungsmittelvorrichtung mit und/oder chemische Stabilisatoren des Zirkonoxid,
- Fig. 12: Einen Querschnitt durch einen porösen, keramischen Körper, eine Verteilungssteuerungsvorrichtung sowie einen Beladungskörper.

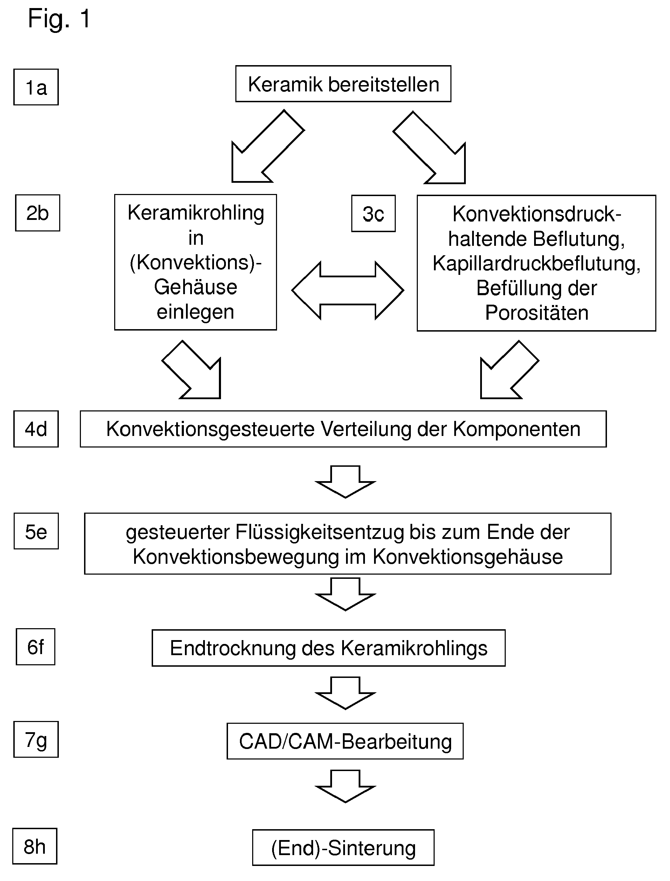

Die Fig.1 stellt ein Ablaufschema des Erfindungsgemäßen dar. A) es werden nur poröse oder und/oder vorgesinterte und/oder entbinderte und/oder gebinderte Keramik für die CAD/CAM Bearbeitung bereitgestellt.
B) Der Keramikkörper wird dann in eine möglichst luftdichtschließende abdichtungsisolierende Form gelegt, z.B. in ein Silikonformgehäuse. Das z.B. Silikongehäuse kann in allen seinen Größen, allen Parametern frei wählbar sein und keinen atmosphärischen Druck haben und/oder einen solchen aufbauen können.
C) Anschließend wird durch luftfreien Beladung der kapillardruckenthaltende Beladungskörperstofflösungsmittelvorrichtung und/oder Farbpigmentlösung und/oder Farbpigmentstabilisatorenlösung und/oder chemischen Stabilisatoren in die Porositäten des porösen Keramikrohlings beladen. Hierzu eignen sich alle Beladungskörperstoffe zur auf oder in der kapillardruckenthaltende Beladungskörperstofflösungsmittelvorrichtung die auch einen kapillardruckgesteuerter Ausgleichstank besitzen können, um Kapillardruck konstant zu halten.
D) Im z.B. Silikongehäuse beginnt jetzt der Verteilungssteuerungsschritt der farbgebenden Komponenten und/oder Farbpigmentlösung und/oder Farbpigmentstabilisatorenlösung und/oder chemischen Stabilisatoren in der porösen Keramik. Das z.B. Silikongehäuse hält oder bildet zudem offene und geschlossene Flächen aus, die mit einer bestimmten Luftfeuchtigkeit umgeben sind, um mit der Bewegungsrichtung der Farbpigmente gewünschte Farbgebungen oder Härtegrade zu erreichen um mit der Bewegungsrichtung und/oder Farbpigmentlösung und/oder Farbpigmentstabilisatorenlösung und/oder chemischen Stabilisatoren die gewünschte Farbgebung und z.B. veränderte Härtegrade und/oder Rissfähigkeit der Oxidkeramik zu erreichen.
E) Flüssigkeitsentzug bis Ende der Verteilungssteuerungsschritt
F) Trocknung der porösen Keramik und/oder zur Kristallbildung und/oder Wärmebehandlung zur Oxidphasenbildung und/oder Kalzinierung und/oder Endsinterung
G) CAD/CAM Bearbeitung unter Berücksichtigung des Schrumpfungsausgleiches
H) sind Sinterung, Fixierung und Umwandlung der Stabilisatoren und farbgebende Komponenten in Oxide zur Stabilisierung und Kalzinierung, sowie die Endsinterung auch in einem Sinterprogramm.

Das bedeutet, dass das z.B. Silikongehäuse mindestens an den Seitenflächen der fräsbaren Keramikrohlings möglichst dicht abschließt, da wie in einem Gefäß eine Konvektionsströmung eingestellt werden kann. In der Stabilisierung und farbgebenden Lösung eingebrachte und nicht farbgebende Komponenten können jetzt gleichmäßig oder im gewünschten Konzentrationsverlauf verteilt werden. Ohne Abdichtung und/oder Isolierung mittels einer Form ist dies aber nicht steuerbar möglich. Es kommt zu Stauungen oder zu nicht eingefärbten Bereichen, wie die EP 235 97 71 lehrt. Durch Vergrößerung und Verkleinerung z.B. Silikongehäuse an gewünscht offenen Flächen, lassen sich unter Temperatur und umgebender unterschiedlicher Luftfeuchtigkeit, mehrfarbige räumliche Farbkonzentrationswellen oder räumliche Farbkonzentrationskonuskegeln einfach im Keramikkörper festlegen, die dann den natürlichen Zahnnachbau bilden können ,sowie das im Verteilungssteuerungsschritt die technisch wichtige Verteilung der chemischen Stabilisatoren die die Härte des natürlichen Zahnaufbaues nachbilden können. Z.B. die EP 29 19 771 und/oder Noritake lehren einen Keramikblock von dunkel nach hell zu schichten und Kronen mit viel Schneide aus der hellen Einstellungszone herauszufräsen. In der Praxis gibt es aber Kronen mit viel Schneide und intensiven Farbkern, der ist jetzt aber in den nur von dunkel nach hell geschichteten Block nicht möglich, weil in der Zone mit viel Schneide keine Dentinfarbe mehr geschichtet ist. Wolz lehrt einen beliebigen Winkel der vertikalen und horizontallen Zahnachse einstellbar und verschiebbar sind und/oder in der Konzentration der 3 räumliche Farbkonzentrationswelle oder dem räumliche Farbkonzentrationskonuskegel um 360° gedreht werden können. So sind erstmalig eine Vielzahl von ästhetischen Möglichkeiten die programmierbar, sowie Farbzonen die zur Verfügung stehen, die einen räumliche Farbverlauf und einstellbare physikalische Parameter durch den Zusatz an chemischen Stabilisatoren haben. Nach Trocknung bilden sich Kristalle z.B. in dotiertem Zirkonoxid. Die Kristalle bilden unter Temperatureinwirkung zu einer Oxidphasenkalzinierung aus. Die zu dotierten Stabilisatoren Oxidationen sind somit sehr gleichmäßig in dem Zr-Gitter verteilt, was wichtig für eine gute physikalische Endsinterung ist.

Die Beladungskörperstoffe können unterschiedliche Konzentrationen nebeneinander besitzen oder können hintereinander gesteuert werden. Durch die kapillardruckenthaltende Beladungskörperstofflösungsmittelvorrichtung werden diese in gewünschte Positionen der Porosität des Keramikrohlings gebracht, die somit gesteuert vollständig die Porositäten belegen können. Es stellte sich heraus, dass bei gleichem Kapillardruck die Farbpigmentlösung und/oder Farbpigmentstabilisatorenlösung und/oder chemischen Stabilisatoren hintereinander oder nebeneinander gehalten werden, ohne sich zu vermischen. Somit ist auch eine Mehrfarbigkeit erreichbar, die durch einen regelbaren Verteilungssteuerungsschritt der Farbpigmente erstmalig z.B. in einem Silikongehäuse wunschgemäß verteilt werden inklusive der Verteilung des chemischen gelösten Stabilisatoren der Zirkonoxidkeramik.

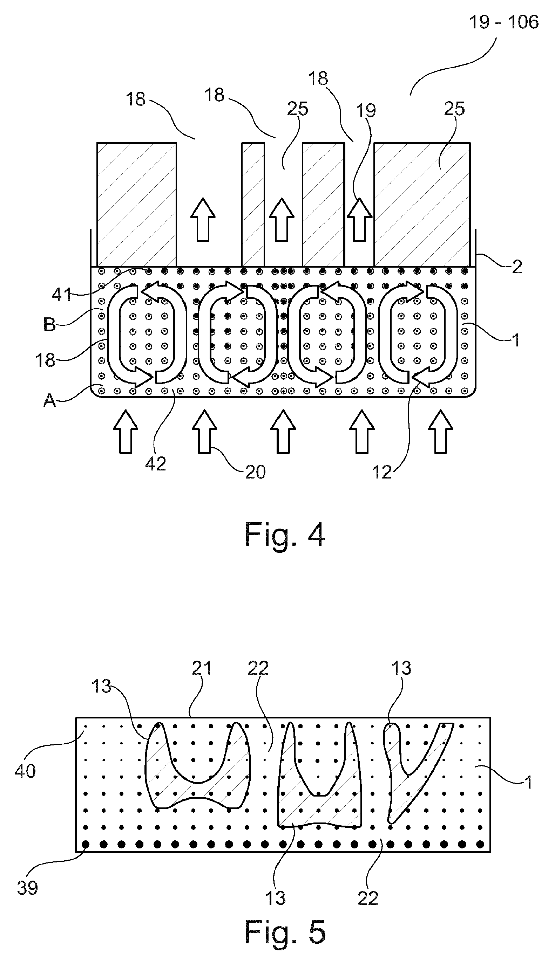

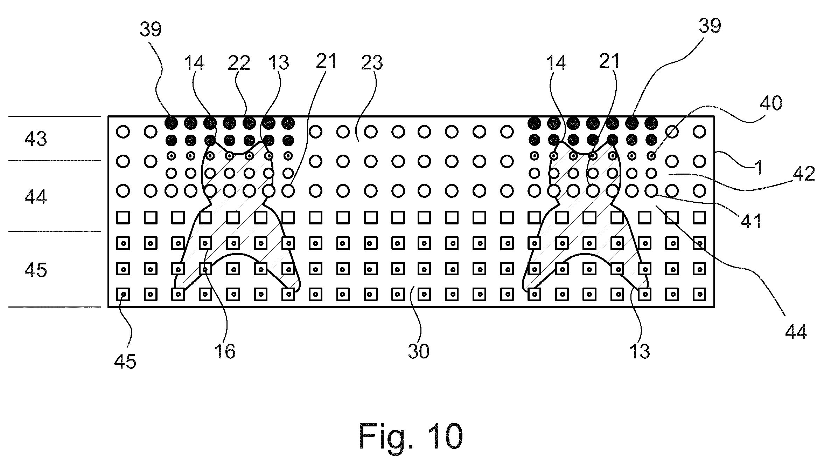

Erfindungsgemäß befindet sich am oder unter dem z.B. Silikongehäuse ein Beladungskörperstofflösungsmittelspeicher, der mit porösen und/oder schwammartigen Stoffen wie z.B. Mikrofaser, Schwämme, Zellstoff usw. gefüllt ist. Der Beladungskörperstofflösungsmittelspeicher sollte doppelt so viel Farbpigmentlösung und/oder Farbpigmentstabilisatorenlösung im Volumen aufnehmen können, wie die bereitgestellte poröse Keramik. Die Beladungskörperstoffe speichern auch farbgebende und nichtfarbgebenden Komponenten wie chemische Stabilisatoren in Lösung , die zu einem Spannungsausgleich von sehr hohem Farbkonzentrationen in nur einem Bereich des keramischen Rohlings beim Endsinterbrand führen, die dann auf der gegenüberliegenden Seite ausgeglichen werden können und/oder die Härte der Zirkonoxidkeramik verringern können z.B. Fig. 5. Des weiteren ist die Herstellung einer Teil- und/oder Totalprothesenrohlings der eine Zahnfarbe, Zahnbogen und einen rosafarbenen Anteil besitzt, die mit der Verteilungssteuerungsschritt regelbar in der z.B. Silikongehäuse gewünschte Farbgebung erreicht, siehe Fig. 10. Die Beladung z.B. mit einer reinen Stabilisatorenlösung durchgeführt werden, um Konzentrationen durch den Verteilungssteuerungsschritt an gewünschte physikalische Zonen des keramischen Rohlings gezielt zu bewegen.

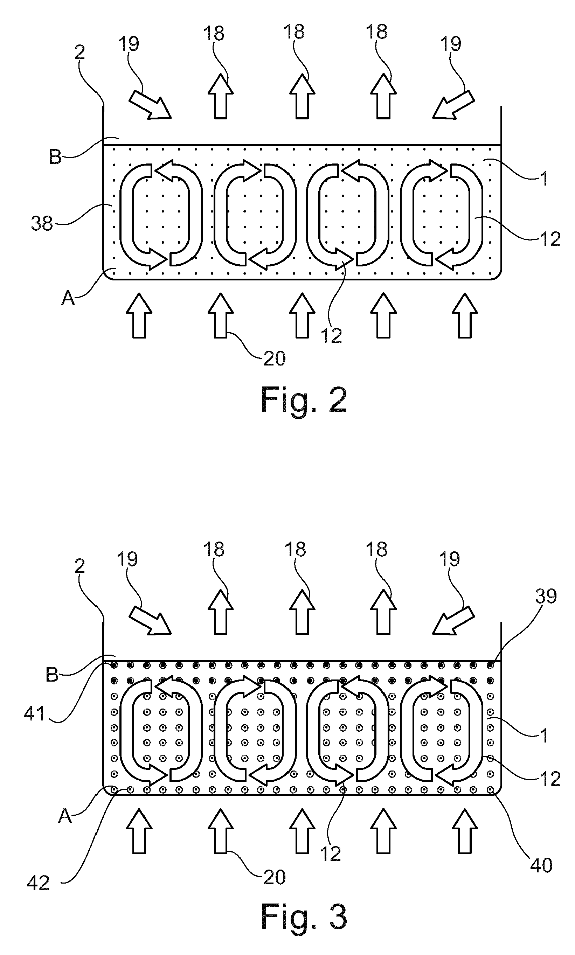

Fig. 2 stellt den Querschnitt eines monochromen porösen Keramikrohlings dar, der mit der kapillardruckenthaltende Beladungskörperstofflösungsmittelvorrichtung beladen wurde. Im z.B. Silikongehäuse wird Flüssigkeit entzogen. Der poröse Keramikrohling ist während der Verteilungssteuerungsschritt der gewünschten Konzentration in dem z.B. Silikongehäuse umgeben. Der poröse Keramikrohling stellt ohne Silikongehäuse einen keramischen Filter dar, bei dem sich bei der Infiltration Konzentrationen ansammelt die nicht gewünscht sind, wie die Lehre der DE 10 2008 026 980 lehrt. In einem z.B. Silikongehäuse lässt sich erstaunlicher Weise aus diesen Konzentrationsansammlungen in einem porösen Keramikrohling eine Bewegungsrichtung der Farbpigmentlösung und/oder Farbpigmentstabilisatorenlösung und/oder chemischen Stabilisatoren verteilend steuernd bewegen. Bei der Herstellung von gleichmäßig verteilten Stabilisatoren und/oder Chromata in keramischen Rohlingen kann man folgender Maßen vorgehen. Mit an gegebenen komplexen Beladungsmittellösungen können sowohl räumliche Polychrome oder monochrome Einfärbungen und Stabilisatorenkomplexen in vorgefertigte keramische Rohlinge beladen werden. Die verschiebende Farben und Chromate sind dem keramischen Fachmann bekannt und in den aufgeführten Schriften beschrieben. Eine Vorauflösung aus Destillat ( Wasser) und 0,05-50% Gewichtsanteil bezogen auf den Feststoffgehalt der Keramik, Aluminiumnitrat oder Yttriumnitrad oder Zirkonnitrad oder Cernitrad oder Polyacryls Salz verwenden, um chemische Stabilisatoren aufzulösen. Auf dem Rollenbock mit ca. 20 Umdrehungen pro Minute 24 Stunden gemischt. Danach erfolgt die Ermittlung des Porositätsvolumens des gewünschten vorgefertigten keramischen Rohlings. Bei der in der Verwendung stehenden DD Biozirkon der Fa. Dental Direkt, ZX 2 Durchmesser 98mm Höhe 14mm 3Y-TzP Chargen Nr. 50143002 Gewicht 330gr, war ein Beladungsvolumen der Porositäten von 50gr Beladungslösungsmittel möglich. Dies ist je nach Hersteller neu zu ermitteln. Nach der Ermittlung können jetzt verschiedene Pigmente der farbgebenden Salze zugegeben werden. Auf 50gr vorgelöster Beladungsmittellösung werden z.B. 1-6gr Erbium und 0,1-1gr FE zugemischt und 1-24 Stunden auf dem Rollenbock gemischt. Die Farbeinstellungen sind abhängig von der Porosität Reinheit der Grundmaterialien und gewünschten Kronzentraionsverlauf des Chromates. Die technischen Anforderungen müssen eingestellt werden. Diese sind in den offenbarten Schriften beschrieben. Auch können alle Mischungen von Pigmentsalzen und Stabilisatoren im beschriebenen Beladungskörperstoffen z.B. Biereckel oder Schaumstoffform mit Farblösung vollständig beladen werden oder nach Beladung getrocknet werden oder ein Beladungssteuerungsschritt kann auch nur mit Stabilisatorenmischung durchgeführt werden. Die farbgebenden Komponenten werden auf Fläche A des porösen Keramischen Rohlings, durch kapillardruckenthaltende Beladungskörperstofflösungsmittelvorrichtung, eine kapillarunterdruck Beladung durchführen, dann um 180° gedreht, wenn das z.B. Silikongehäuse keine verstellbaren Abdeckungen hat siehe Fig. 2. Alle Farbkomponenten 4 wandern zur Fläche B des porösen keramischen Rohlings nach ca. 80%-90% Flüssigkeitsentzug unter 70% Luftfeuchtigkeit an Fläche B wird nun wieder um 180° gedreht, wenn Konvektionsgehäuse keine verstellbaren Abdeckungen hat. Somit findet eine homogene Verteilung der Konzentration statt, da die Flüssigkeit entzogen wird, bevor sich wieder Farbkonzentrationen und/oder Farbpigmentstabilisatorenlösung und/oder chemischen Stabilisatoren Konzentrationen aus der Lösung der chemischen Stabilisatoren bilden können. Die Angaben sind abhängig von eingestellter Viskosität der Farbpigmentlösung und/oder Farbpigmentstabilisatorenlösung und Porosität des keramischen Rohlings.

Fig. 3 stellt den Querschnitt eines polychromen porösen Keramikrohling dar bei dem ein farbgebender Konzentrationsverlauf und Stabilisatoren von dunkel nach hell hergestellt wird. Fläche B wird mit kapillardruckenthaltende Beladungskörperstofflösungsmittelvorrichtung beladen und im z.B. Silikonform Flüssigkeit entzogen. Die farbgebenden Komponenten können unter Kapillardruck stehen, in den Beladungskörperstoffe hintereinander, übereinander oder nacheinander eingebracht werden, je nach Anspruch des Farbwunsches oder der technisch gewünschten Parameter je nach Anspruch des Farbwunsch oder technisch gewünschte physikalische Parameter. Eine einfache Kapillarunter-druckbeladung mit einer farbkompetenten Lösung erzeugt natürlich auch einen verteilungsgesteuerten Farbverlauf von dunkel nach hell der Fläche B, die mit einer Luftfeuchtigkeit von 30%-80% in Berührung steht. Man hat herausgefunden, dass z.B. 50% Luftfeuchtigkeiteine stärkere Bewegungs-richtung der Farbpigmente und/oder chemischen gelösten Stabilisatoren im Verlauf entsteht. Also mehr dunkel und mehr hell und bei 80% Luftfeuchtigkeit eine leichte d.h. weniger dunkel - weniger hell Bewegungsrichtung der Farbpigmente und/oder Stabilisatoren von dunkel nach hell. Somit sind in Zonen mit dunklen Konzentrationsansammlungen auch mehr Stabilisatorenkonzentrationen aufzufinden. Die Stabilisatorenkonzentrationen können somit auch durch die Farbpigmente sichtbar gemacht werden. Der poröse Keramikrohling ist bis zur nicht mehr Bewegbarkeit der Komponenten Flüssigkeiten zu entziehen. Die Trockenzeit ist abhängig von Porosität und Größe des porösen keramischen Rohlings und der Luftfeuchtigkeit, Raumtemperatur und damit verbundenen gewünschten Komponenten Konzentrationen.

Fig. 4 stellt den Querschnitt eines polychromen Keramikrohlings dar, der dazu noch dreidimensionalen Farbzonen aufweist. Fläche B wird mit kapillardruckenthaltende Beladungskörperstofflösungsmittelvorrichtung und in Konvektionsgehäuse mit entsprechenden möglichst luftdichten Flächen und/oder Rahmen auf Fläche B gedrückt und Flüssigkeit entzogen. Zur Unterstützung von kapillardruckenthaltende Beladungskörperstofflösungsmittelvorrichtung können auch Beladungskörperstoffe Hilfsstoffe wie Fig.8 und Fig.9 als Beladungskörperlösungsmittelspeicher sein, die kapillardruckenthaltende Beladungskörperstofflösungsmittelvorrichtung unterstützen. Erstaunlicher Weise ist die ersichtliche Bewegungsströmung so stark, das auch eine einfache kapillardruckenthaltende Beladungskörperstofflösungsmittelvorrichtung mit einer farbgebenden und/oder einer Stabilisationskomponenten Lösung des porösen Keramikrohlings, der nach der Beladung z.B. mit Tesastreifen abgeklebt, um Öffnungen oder abdeckende Flächen zu erzeugen. Der Verteilungssteuerungsschritt kann so stark sein, dass aus allen Bereichen die mit farbgebenden Komponenten in den porösen Keramiken gefüllt sind, diese zu den offenen Flächen wandern.

Fig.5 stellt einen Querschnitt eines porösen Keramikrohlings dar, der dazu noch dreidimensionale physikalische Zone aufweist und getrocknet ist. Der physikalische Farbverlauf der Zonen wird graphisch auf einer Software dargestellt (13). Der Zahntechniker ganz selbst oder an Hand von digitaler physikalischen Daten den gewünschten Zahnfarbenkonzentrations- und Konzentrationsverlauf (21) (22) (39) (40) aussuchen. Die CAD/CAM Anlage fräst dann aus gewünschten Zonen des keramischen Rohlings(1) den Zahnersatz(13) heraus.

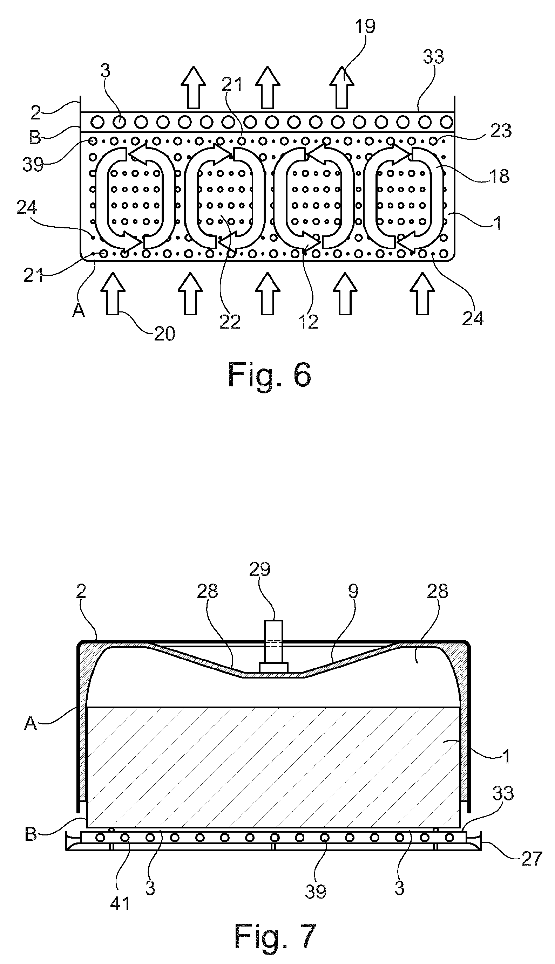

Fig.6 Stellt einen Querschnitt eines porösen Keramikrohlings dar, der schon getrocknet ist. Der eine starke Farbkonzentrationskomponenten (21) im Flächenbereich A aufweist. Bei der Endsinterung je nach Porosität und Herstellungsverfahren der keramischen Rohlingen (1) kann es zu Spannungen des endgesinterten keramischen Rohlings kommen. Wenn diese nicht durch das Herstellungsverfahren des porösen keramischen Rohlings ausgeglichen werden können. Gibt man einfach, wie in Fig. 6, auf einen schon getrockneten porösen keramischen Rohling einen Beladungskörperstoff mit dem entsprechenden nicht farbgebenden Komponenten und/oder chemische Stabilisatoren mit physikalisch gewünschten Eigenschaften mit einer Kapillarunterdruckbeladung dazu. Es wird die Konzentration berechnet und mit nicht farbgebenden Komponenten zu wie z.B. Yttriumnitrat, Aluminiumnitrat, Cernitrat, Zirkoniumnitrat, Kaliumnitrat, Caliciumnitrad, Zinknitrat und Lanthannitrat ausgeglichen. Um gleichzeitig z.B. die Härte im Occlusionsbereich (Kaufläche im Zahnbereich) zu verringern. Es kann aber auch durch Berechnungen der Beladungskörperstoffe erfolgen. Die unter Kapillardruck nacheinander, übereinander in die poröse Keramik kapillardruckenthaltende Beladungskörperstofflösungsmittelvorrichtung eingebracht werden.

Fig.7 Stellt einen Querschnitt der kapillardruckenthaltende Beladungskörperstofflösungsmittelvorrichtung in einer z.B. Silikonformgehäuse dar. Der poröse keramische Rohling wird in die z.B. Silikonformgehäuse gedrückt, mit Ventil oder Stempel atmosphärischer Druck verringert. Der poröse keramische Rohling wird auf Fläche B auf trockene und/oder unter Kapillardruck stehende Beladungskörperstoffe der ein Beladungskörperlösungsmittelspeicher ist, eine Kapillarunterdruckbeladung erzeugt. Die Kapillarräume eines 14mm hohen Keramikrohlings mit einem Durchmesser von 98mm benötigen ca. 50gr an Farbpigmentlösung und/oder Farbpigmentstabilisatorenlösung und/oder chemische Stabilisatoren zur Kapillarraumbefüllung, die durch eine Kapillarunterdruckbeladung im z.B. Silikongehäuse in ca. 25 min erreicht wird. Die Zeit ist aber abhängig von Farbpigmentlösung und/oder Farbpigmentstabilisatorenlösung und/oder chemische Stabilisatoren der Konzentrierung der eingestellten Viskosität der Kapillardruck Unterstützung durch Verhältnisse in der Silikongehäuse und der Art des Materials und Größe der Porositäten.

Fig.8 stellt einen Querschnitt von Beladungskörperstoffen dar, die farbgebende oder nicht farbgebende Komponente und Stabilisatorenkomponenten in einer Kapillarunterdruckbeladung in der porösen Keramik erzeugen, dabei stehen die Beladungskörperstoffe unter gleichem Kapillardruck. So kommt es zu keiner Vermischung unterschiedlichen Farbkomponenten und/oder nicht farbgebenden Komponenten und/oder nicht farbgebenden Komponenten und/oder Lösungen der chemischen Stabilisatoren. Somit können nebeneinander oder hintereinander oder übereinander alle möglichen Farbgebungen und Spannungsausgleiche physikalische Eigenschaften durch Farbpigmentlösung und/oder Farbpigmentstabilisatorenlösung in die poröse Keramik beladen werden. Ganz wichtig ist auch das jegliche farbgebende Form Konturen wie z.B. Kieferform, einzelne Zahnformen, Implantate, Abbumentformen, in horizontal oder vertikalem Querschnitt aus Beladungskörperstoffen ausgeschnitten, gefräst, gestanzt oder geplottet usw. hergestellt werden können. Sowie das Beladungskörperstoffe Kapillarvolumen des Konzentrationsverlaufes auf das Kapillarvolumen der porösen Keramik berechenbar sind.

Fig.9 stellt den Querschnitt von fünf Beladungskörperstoffen geschichtet dar, um dreidimensionale Prothesenkörper herzustellen. Z.B. als werden fünf Beladungskörperstoffe übereinander gelegt. Dazu eignen sich 1,4mm starke Bierdeckel im Durchmesser 104mm diese speichern ohne Probleme über 10g an Farbpigmentlösung und/oder Farbpigmentstabilisatorenlösung und/oder farbgebender Komponenten und/oder Lösungen der chemischen Stabilisatoren des Zirkonoxid. Das bedeutet, dass die benötigte Farbpigmentlösung und/oder Farbpigmentstabilisatorenlösung und/oder Lösungen der chemischen Stabilisatoren Volumen in fünf Beladungskörperstoffe ohne Vermischung in die porösen keramischen Rohlinge unter Kapillarunterdruckbeladung auf der kapillardruckenthaltende Beladungskörperstofflösungsmittelvorrichtung durchgeführt. In dem z.B. Silikongehäuse wird nun die Flüssigkeit entzogen. Die Flüssigkeitsentzugsgeschwindigkeit pro 1,0mm der porösen Keramik beträgt dann 24 Stunden, je nachdem wie die Farbpigmentlösung und/oder Farbpigmentstabilisatorenlösung und/oder Lösungen der chemischen Stabilisatoren zusammengesetzt und hergestellt wurde und/oder die Umgebung der Luftfeuchtigkeit von 50%-90% eingestellt ist.

Fig.10 stellt einen Querschnitt eines getrockneten porösen keramischen Rohlings dar, der mit polychromen räumlichen physikalischen Zonen und Farbverlauf aufweist. Der Farbverlauf des gesamten Prothesenkörpers wir graphisch von der Software dargestellt, Der Zahntechniker bestimmt selbst oder auf Grund digitaler Farbdaten den gewünschten Prothesenkörper Zahnfarbenverlauf und physikalische wichtige Zonen. Die CAD/CAM Anlage fräst dann die gewünschten Prothesenkörper mit entsprechenden Farbverlauf und Härtegrad und Biegefestigkeitwerte aus dem porösen keramischen Rohling heraus.

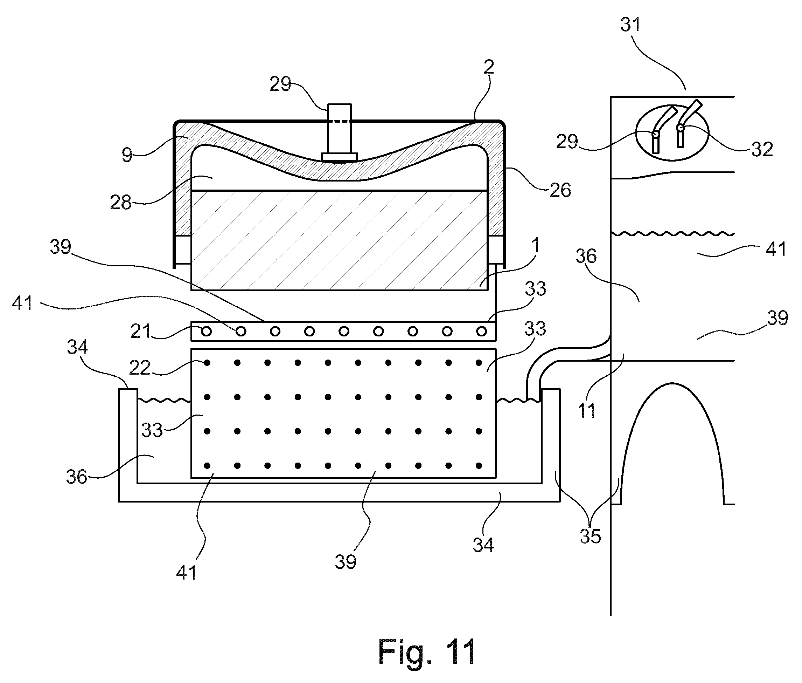

Fig. 11 stellt den Querschnitt des kompletten Systems dar, mit z.B. Silikonformgehäuse und porösen Keramikrohlingen (2) austauschbaren oder schichtbaren Beladungskörperstoffe (33) mit möglichen Farbkomponenten Farbpigmentlösung und/oder Farbpigmentstabilisatorenlösung und/oder Lösungen der chemischen Stabilisatoren der Oxidkeramik unter Kapillardruck(21) (41). Kapillardruckenthaltende Beladungskörperstofflösungsmittelvorrichtung und Speicher aus (7) (porösen oder schaumartigen Stoffen) mit und/oder ohne farbgebenden Komponenten als Speicher mit möglichen kapillardruckgesteuerter Ausgleichstank (31), mit unter atmosphärischen Druckventil und/oder Unterdruckaufschlagungsventil (29).

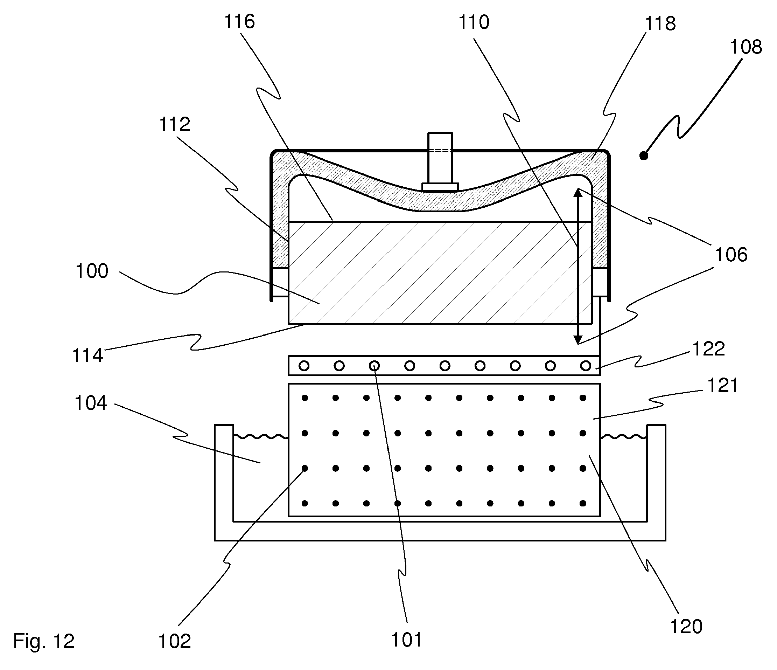

Gemäß Figur 12 ist der poröse, keramische Körper 100 in eine Form 118, insbesondere eine Silikonform formschlüssig eingepasst, wobei eine frei zugängliche Oberfläche 114 zur Beladung mit chemischen Stoffen 101, 102, die zur Beeinflussung der physikalischen Eigenschaften des porösen, keramischen Körpers 100 geeignet sind und in einer Lösung 104 enthalten sind, auf einen Beladungskörper 120 mit zwei Schichten 121, 122 auflegbar. Bezüglich der frei zugänglichen Oberfläche 114 und einer isolierten und/oder abgedichteten Oberfläche 116 ist ein Umgebungsparametergradient 110 zwischen einem Parameter 105 innerhalb der Form 118 und einem Umgebungsparameter 106 in einer Umgebung 108 durch Regelung des Umgebungsparameters 106 und/oder Regelung des Parameters 105 einstellbar.

### Bezugszeichenliste

1 - poröse und/oder gesinterte und/oder entbinderte und/oder gebinderte Keramik bereitstellen
2 - z.B. Silikonform mit eingelegtem porösen Keramikrohling,
3 - Beladung der Porositäten des Keramikrohlings, wobei die Schritte 2 und 3 in beliebiger Reihenfolge ausgeführt werden können
4 - Verteilungsschritt der Komponenten
5 - Flüssigkeitsentzug bis Ende des Verteilungssteuerungsschrittes in z.B. Silikonform
6 - Trocknung des porösen Keramikrohlings
7 - CAD/CAM Bearbeitung
8 - Innenfläche des Zahnersatzes und/oder des Implantats oder Implantatprothetik
9 - Silikon
10- Ventil
11 -
12 - Zeichen für Verteilungssteuerungsschritt
13 - gestrichelte Bilder zur geplanten CAD/CAM Bearbeitung
14 - Prothesenzahn
15- rosafarbener Farbanteil
16 - mögliche Einlagerung in 3 Dimensionale Farbraum
17- Farbkomponenten
18 - Flüssigkeitsentzug
19- Luftfeuchtigkeit
20 - Temperatur
21 - farbgebende Komponenten Konzentrationen (groß)
22 - farbgebende Komponenten Konzentrationen (klein)
23 - farblose Komponenten z.B. zum Konzentrationsausgleich groß
24 - farblose Komponenten z.B. zum Konzentrationsausgleich klein
25 - z.B. Silikongehäuseabdeckrahmen
26 -
27 - Gefäß
28 - unter atmosphärischen Druckbereich oder ohne atmosphärischen Druck
29 - unter atmosphärischen Druck auslösendes Ventil
30 - Zahnfarbe rosa Zahnfleischfarbe
31 - Kappilardruck gesteuerter Ausgleichtank
32 - Druckeinstellventile
33 - Beladungskörper
34 - kapillardruckenthaltende Beladungskörperstofflösungsmittelvorrichtung
35 - Beladungskörperstofflösungsmittespeicher
36 - Farbpigmentlösung
37 - Stabilisatoren
38 - Stabilisatoren vor Kalzinierung
39 - Härte senkende Stabilisatoren Komponenten Konzentrationen (groß)
40 - Härte senkende Stabilisatoren Komponenten Konzentrationen (klein)
41 - Stabilisatoren und/oder farbgebende Konzentrationen (groß)
42 - Stabilisatoren und/oder farbgebende Konzentrationen (klein)
43 - Zone C = Härte HV von 450 bis 1450
44 - Zone D = Biegefestigkeit MPa von 500 bis 2000
45 - Zone E = Bruchzähigkeit in MPa/m² von 5-16
a,b,c,d Einfärbung mit Abstufungen von hell bis dunkel
110- poröse Keramik
101, 102 - chemische Stoffe, die zur Beeinflussung physikalischer Eigenschaften geeignet sind
104- Lösung
105- Parameter
106- Umgebungsparameter
108- Umgebung
110 - Umgebungsparametergradient
112- isolierter und/oder abgedichteter Teil einer Oberfläche
114 - zweite, frei zugängliche Oberfläche
116 - erste , isolierte und/oder abgedichtete Oberfläche
118 - Form
120 - Beladungskörper
121, 122 - Schicht

## Patentansprüche

1. Verfahren zur Herstellung eines keramischen Körpers (100), insbesondere eines Dentalkeramikrohlings, mit gezielt einstellbaren Ausprägungsgraden einer oder unterschiedlicher physikalischer Eigenschaften, **dadurch gekennzeichnet, dass** der keramische Körper (100) zur Steuerung einer gezielten Verteilung eines oder verschiedener chemischer Stoffe (101, 102), die zur Beeinflussung der physikalischen Eigenschaften des keramischen Körpers (100) geeignet sind, eine Porosität aufweist, und in einem ersten Schritt, der ein Beladungsschritt ist, mit einer oder mit mehreren Lösungen (104) des einen oder der verschiedenen chemischen Stoffe (101, 102) beladen wird, und in einem zweiten Schritt, der ein Verteilungssteuerungsschritt ist, die Verteilung des einen oder der verschiedenen chemischen Stoffe (101, 102) innerhalb des porösen, keramischen Körpers (100) gesteuert wird, wobei ein Verlauf und/oder ein räumlicher Verlauf des Ausprägungsgrads der einen oder der unterschiedlichen physikalischen Eigenschaften erzeugbar ist, und wobei die Steuerung durch Regelung eines oder mehrerer Umgebungsparameter (106) in einer Umgebung (108), insbesondere durch Regelung der Luftfeuchtigkeit und/oder des Drucks und/oder der Temperatur erfolgt.

2. Verfahren nach Anspruch 1, **dadurch gekennzeichnet, dass** die Verteilung des einen oder der verschiedenen chemischen Stoffe (101, 102) innerhalb des keramischen Körpers (100) durch eine Konvektionsströmung bewirkt wird, wobei eine Strömungsrichtung und -geschwindigkeit durch gezieltes Erzeugen von Umgebungsparametergradienten (110), insbesondere durch Einstellen von Luftfeuchtigkeitsunterschieden und/oder Druckunterschieden und/oder Temperaturunterschieden, bezüglich verschiedener Oberflächen (116, 114) der porösen Keramik (100) gesteuert werden.

3. Verfahren nach einem der vorhergehenden Ansprüche, **dadurch gekennzeichnet, dass** eine Bewegungsgeschwindigkeit des einen oder der verschiedenen chemischen Stoffe (101, 102) und/oder die Strömungsgeschwindigkeit durch Erhöhen und/oder Verringern eines oder mehrerer Umgebungsparametergradienten (110) gesteuert wird.

4. Verfahren nach einem der vorhergehenden Ansprüche, **dadurch gekennzeichnet, dass** eine Bewegungsrichtung des einen oder der verschiedenen chemischen Stoffe (101, 102) und/oder die Strömungsrichtung durch Änderung der Richtung eines oder mehrerer Umgebungsparametergradienten (110) gesteuert wird.

5. Verfahren nach einem der vorhergehenden Ansprüche, **dadurch gekennzeichnet, dass** mindestens eine Oberfläche (116) oder mindestens ein Teil einer Oberfläche (112) des keramischen Körpers (100) während des Beladungsschritts und/oder während des Verteilungssteuerungsschritts isoliert und/oder abgedichtet ist und, dass mindestens eine andere Oberfläche (114) oder mindestens ein anderer Teil einer Oberfläche des keramischen Körpers (100) für die Beladung und/oder für die Steuerung frei zugänglich ist.

6. Verfahren nach einem der vorhergehenden Ansprüche, **gekennzeichnet durch** die nachfolgenden Schritte,
- Bereitstellung eines porösen Keramikrohlings (100),
- Versehen einer oder mehrerer Oberflächen (112, 116) des Keramikrohlings (100) mit einer Isolierung und/oder Abdichtung, wobei der Keramikrohling passgenau innerhalb einer flüssigkeits-/luftundurchlässigen Form (118), insbesondere einer Silikonform platziert wird, sodass mindestens eine Oberfläche (114) des Keramikrohlings (100) frei zugänglich ist,
- Beladen der frei zugänglichen Oberfläche (114) des Keramikrohlings (100) mit einem oder verschiedenen chemischen Stoffen (101, 102), wobei der eine oder die verschiedenen chemischen Stoffe (101, 102), die zur Beeinflussung der physikalischen Eigenschaften des Keramikrohlings (100) geeignet sind in einer oder mehreren Lösungen (104) enthalten sind,
- Platzieren des Keramikrohlings (100) innerhalb einer Umgebung (108), deren Umgebungsparameter (106), insbesondere die Luftfeuchtigkeit und/oder der Druck und/oder die Temperatur, regelbar sind, wobei die frei zugängliche Oberfläche (114) des Keramikrohlings (100) mit der Umgebung (108) in Verbindung steht,
- Steuerung der Verteilung des einen oder der verschiedenen chemischen Stoffe (101, 102) innerhalb des Keramikrohlings (100), wobei mindestens ein Umgebungsparameter (106), insbesondere die Luftfeuchtigkeit und/oder der Druck und/oder die Temperatur zur Erzeugung eines Umgebungsparametergradienten (110) zwischen der frei zugänglichen Oberfläche (114) und der einen oder den mehreren isolierten und/oder abgedichteten Oberflächen (112, 116) des Keramikrohlings (100) geregelt wird.

7. Verfahren nach einem der vorhergehenden Ansprüche, **dadurch gekennzeichnet, dass** die eine oder die mehreren Lösungen (104) eines oder verschiedener chemischer Stoffe (101, 102) derart innerhalb des keramischen Körpers (100) gesteuert werden, dass eine Konzentration der gelösten chemischen Stoffe (101, 102) in verschiedenen Bereichen des keramischen Körpers unterschiedliche Werte aufweist, sodass in verschiedenen Bereichen des keramischen Körpers (100) unterschiedliche Ausprägungsgrade der physikalischen Eigenschaften eingestellt werden.

8. Verfahren nach einem der vorhergehenden Ansprüche, **dadurch gekennzeichnet, dass** die Lösung (104) destilliertes Wasser, Zirkonnitrat sowie mindestens einen chemischen Stoff (101, 102), der zur Beeinflussung der physikalischen Eigenschaften des keramischen Körpers (100) geeignet ist, umfasst.

9. Verfahren nach einem der vorhergehenden Ansprüche, **dadurch gekennzeichnet, dass** ein Ausprägungsgrad einer Opazität und/oder einer Transluzenz des keramischen Körpers (100) mittels einer Yttrium enthaltenden Lösung (104) gesteuert wird.

10. Verfahren nach einem der vorhergehenden Ansprüche, **dadurch gekennzeichnet, dass** ein Ausprägungsgrad einer Härte und/oder einer Festigkeit und/oder einer Zähigkeit des keramischen Körpers (100) mittels einer Cer enthaltenden Lösung (104) gesteuert wird.

11. Verfahren nach einem der vorhergehenden Ansprüche, **dadurch gekennzeichnet, dass** eine Konfiguration eines Kristallsystems des keramischen Körpers (100) oder einzelner Bereiche des keramischen Körpers (100) mittels einer Calcium und/oder Magnesium und/oder Yttrium enthaltenden Lösung (104) gesteuert wird.

12. Verfahren nach Anspruch 11, **dadurch gekennzeichnet, dass** das Kristallsystem des keramischen Körpers (100) zumindest bereichsweise in einer kubischen Konfiguration stabilisiert wird, wobei die Lösung (104) einen Molanteil von mindestens 16 % Calcium und/oder 16 % Magnesium und/oder 8 % Yttrium enthält.

13. Verfahren nach einem der Ansprüche 11 oder 12, **dadurch gekennzeichnet, dass** das Kristallsystem des keramischen Körpers (100) zumindest bereichsweise in einer tetragonalen Konfiguration stabilisiert wird, wobei die Lösung (104) einen Molanteil von mindestens 8 % Calcium und/oder 8 % Magnesium und/oder 4 % Yttrium enthält.

14. Verfahren nach einem der vorhergehenden Ansprüche, **dadurch gekennzeichnet, dass** ein Kristallsystem innerhalb von Poren des keramischen Körpers (100), mittels einer Lösung (104), die Zirkonnitrat sowie Calcium und/oder Magnesium und/oder Yttrium umfasst, in einer kubischen und/oder tetragonalen Konfiguration stabilisiert wird.

15. Keramikrohling (100), geeignet zur Herstellung von Zahnersatz mittels einer CAD/CAM-Fräsmaschine, insbesondere in einem Verfahren, nach einem der Ansprüche 1-14 hergestellt, **gekennzeichnet durch** eine räumliche Verteilung eines oder verschiedener chemischer Stoffe (101, 102), die zur Beeinflussung der physikalischen Eigenschaften des Keramikrohlings (100) geeignet sind, wobei die räumliche Verteilung des einen oder der verschiedenen chemischen Stoffe (101, 102) mittels Umgebungsparametergradienten (110) steuerbar ist, und wobei der Keramikrohling (100) zur Einstellung eines graduellen und/oder abgestuften Verlauf des Ausprägungsgrades einer oder mehrerer unterschiedlicher physikalischer Eigenschaften einer Wärmebehandlung, insbesondere einem Sintervorgang unterziehbar ist.
